# EUROPÄISCHE PATENTSCHRIFT

(11) **EP 3 666 294 B1**
(45) Veröffentlichungstag und Bekanntmachung des Hinweises auf die Patenterteilung: **29.04.2026**
(21) Anmeldenummer: 19214090.3
(22) Anmeldetag: 06.12.2019
(51) Int. Cl.: A61L 15/28, A61L 15/32

(54) **PORÖSER KÖRPER AUF BIOPOLYMERBASIS MIT FLEXIBLER PORENGRÖSSE, SEINE HERSTELLUNG UND ANWENDUNG**
POROUS OBJECT BASED ON BIOPOLYMERS HAVING A FLEXIBLE PORE SIZE, ITS PREPARATION AND USE THEREOF
OBJECT POREUX A BASE DE BIOPOLYMERES AVEC UNE GRANDEUR DE PORE FLEXIBLE, SA PREPARATION ET SON USAGE

(30) Priorität: 13.12.2018 DE 102018009814
(43) Veröffentlichungstag der Anmeldung: 17.06.2020
(73) Patentinhaber: Valeopharm GmbH, 22339 Hamburg (DE)
(72) Erfinder: Teslenko, Alexander, 58095 Hagen (DE)
(74) Vertreter: Braeuning Schubert Patentanwälte GbR

(56) Entgegenhaltungen:
- EP-A1- 0 747 420
- WO-A1-2005/063311
- DE-A1- 4 119 140
- US-A- 5 840 777
- BARANWAL ANUPRIYA ET AL: "Chitosan: An undisputed bio-fabrication material for tissue engineering and bio-sensing applications", INTERNATIONAL JOURNAL OF BIOLOGICAL MACROMOLECULES, ELSEVIER BV, NL, vol. 110, 13 January 2018 (2018-01-13), pages 110 - 123, XP085363578, ISSN: 0141-8130, DOI: 10.1016/J.IJBIOMAC.2018.01.006

## Beschreibung

### Gegenstand der Erfindung

Die Erfindung betrifft poröse Körper (PK) auf Basis einer Mischung aus Eiweiß- und Polysaccharid-Naturpolymeren, insbesondere Chitosan, mit weiterhin mindestens einer Mono- und/oder Polycarbonsäure, einem nicht ionischen Tensid und vorzugsweise einem Vernetzer, wobei der PK einstellbare hexagonale oder ovale Porenstrukturen im Bereich von 0,1 µm bis 1000 µm aufweist.

Die Erfindung auch ein Verfahren zu ihrer Herstellung, wobei die PK aus verschäumten wässrigen Zubereitungen auf Basis von Polysacchariden wie vor allem Chitosan und Eiweißen wie vor allem Kollagen, Gelatine entstehen und durch nachfolgende konventionelle beispielsweise thermische Schaum-Trocknung ohne Gefriertrocknung erzeugt werden. Gegebenenfalls kann in der wässrigen Lösung (homogen) oder im getrockneten Produkt (heterogen) eine Vernetzung erfolgen. In Korrelation mit den wählbaren Komponenten und Bedingungen bei der Herstellung - insbesondere Polymere, Verschäumung, Trocknung- können die erzeugten Poren des erhaltenen Produktes im Mikrobereich (Durchmesser ≤ 1,0 µm) oder Makrobereich (Durchmesser größer als 1,0 µm) liegen. Die physikalischen und morphologischen Charakteristiken der Produkte (PK) vor allem die Porengröße, sind somit auf einfache Weise anwendungsbezogen flexibel einstellbar.

Aufgrund dieser Vorgehensweise wird eine kontinuierliche, kostengünstige, reproduzierbare, umweltfreundliche industrielle Herstellung von maßgeschneiderten PK ermöglicht.

Die erfindungsgemäßen PK können eine beachtliche Menge (bis 100 Mal des eigenen Gewichts) an Wasser oder biologischen Lösungen aufnehmen. Dabei wandeln sie sich in bioverträgliche biomimetische 3D-Hydrogele mit Ähnlichkeit zu einer Extrazellulären Matrix um.

Aufgrund der verbesserten mechanischen Eigenschaften sowie der ausgezeichneten Biokompatibilität wie u.a. Haut- /Mucosadhäsion und Penetrationsförderung eignen sich die PK vor allem als Abgabesystem für Wirkstoffe wie kosmetische, oder medizinische Wirkstoffe (Arzneimittel), für die topische kosmetische Behandlung von Haut wie Altershaut, Narben, Cellulite, oder Problem haut, bei Hautkrankheiten wie Psoriasis oder Rosacea oder auch für die topische therapeutische Anwendung z. B. Wundheilung oder Implantierung. Dazu werden die PK auf das zu behandelnde Areal aufgelegt, umwickelt oder auf ähnliche Weise geeignet appliziert.

Die erfindungsgemäßen Produkte eignen sich ferner als Gerüste/Scaffolds für Zellkultivierung und Tissue Ingineering. Sie sind daher anwendbar in den Bereichen regenerative Medizin u.a. für Bioprothetik (Implantate und Prothese), Wundbehandlung oder Zahnmedizin.

### Hintergrund

Poröse Körper (PK) finden großen und wachsenden Einsatz in verschiedenen Gebieten der Life Science und Medizin, u.a. in der Zellkultivierung, Tissue Engineering, Regenerativen Medizin, Bioprothetik (als Implantate und Prothesen), bei therapeutischem Drug Delivery, Gentherapie, Wundmanagement, Hautbehandlungen und Hautpflege.

PK sind definiert als poröse Festkörper, als dünne poröse Festkörper (Membranen), als vernetzte (strukturierte) Körper und Umwandlungsprodukte hiervon wie 3D-Gele (u.a. Hydrogel). Für die Zellkultivierung, Tissue Engeeniering, Bioprothetic oder Wundmanagement am besten geeignet sind makroporöse Körper, dagegen werden für die Arzneimittelabgabe mikroporöse PK benutzt.

### Stand der Technik

In EP 1 696 971 B1 (WO2005/06331) werden Wundauflagen aus Chitosan und Gelatine beschrieben, welche mehrfunktionelle Polycarbonsäuren, polyfunktionelle Aminosäuren und Vernetzer wie Glytardialdehyd, aufweisen. Mittels Gefriertrocknung entstehen Wundauflagen mit relativ homogener Porenstruktur und Porengröße. Eine Steuerbarkeit von morphologischen Parametern hiervon sind nicht beschrieben.

In US 20160243505 A1 werden vernetzte hydrophile biodegradierbare Schaummembrane auf Basis von Algenpolysacchariden beschrieben. Die Herstellung von 3D-porosen Strukturen erfolgt durch Lyophylisierung.

Eine andere herkömmliche Methode bei der Herstellung von PK ist das sog. Templating untert Anwendung sog. Platzhalter oder Porenbildner/Porogenen. Ein Porenbildner ist ein mikronisierter Stoff (Mikropartikel oder Gasbläschen), verteilt in einem Polymermatrix/ Gel, der nach der Trocknung und darauf folgender Entfernung aus einem vernetzten Körper die gewünschten Poren hinterlässt. Für biomedizinische Anwendungen werden Zubereitungen auf Biopolymerbasis mit Porenbildnern (Porogenen) versetzt, wie z. B. durch Einsatz von chemisch generiertem oder überkritischem CO₂, oder nicht gasbildenden Porogenen wie z. B. Salzen, Zuckern, Lösungsmitteln oder Nano/Mikropartikeln, Gelatinesphären oder Paraffinkugeln.

Die Herstellung von makroskopischen Membranen aus Hydrogelen mit Porenbildnern oder Porogenen ist in US20070237811, US 2017/0224868, US5993661, US 7094,372 beschrieben. Typischerweise beträgt die von dem hier verwendeten Porogensystem(en) erzeugte Porengröße etwa 50 bis etwa 1.500 Mikrometer.

US 5840777 A beschreibt ein Verfahren zur Herstellung eines Polysaccharidschaumes durch Einführen von Gas und einem wasserunlöslichen Carbonatsalz (Porogenbehandlung) in eine wässrige Lösung des Polysaccharids.

In EP 0747420 A1 wird ebenfalls eine Methode zur Gewinnung eines Polysaccharidschaumes offenbart, wobei hier eine wässrige Lösung des Polysaccharids mit Gas, vorzugsweise auch weiteren Porogenen, versetzt und schließend sogleich getrocknet wird.

Die DE 41 19 140 A1 betrifft einen in Wasser und Körperflüssigkeit leicht löslichen Formkörper, der aus einer Mischung aus hydrolisiertem Kollagen und nicht abgebauter Gelatine besteht. Die Zubereitung kann zur Erhöhung der Benetzbarkeit polyethoxylerte Tenside aufweisen. Die Mischung wird erhitzt, abgekühlt, abgefüllt und getrocknet, z.B. konvektiv, vorzugsweise lyophilisiert. Wesentlich ist hier das Mischungsverhältnis der Eiweiße, wodurch die gewünschte hohe Auflösungsgeschwindigkeit gesteuert wird.

EP 2 340 002 B1 betrifft eine Wundauflage aus Chitosan, hergestellt mittels mechanisch erzeugten Gasblasen sowie einem Tensid wie Benzalkoniumchlorid, welches nach Gefriertrocknung eine lamellare poröse Struktur aus gleichmäßig verteilten Spuren von Gasblasen aufweist.

In DE102016102782 A1 werden zweischichtige nanoporöse Dialysemembranen mit Hilfe von porenbildenden hydrophilen Additiven aus Polyvinylpyrrolidon mit Molmasse von 1 bis 2,2 kDa oder kurzkettigen Glykolen hergestellt. Die Herstellung Erfolg durch Fällungsmittelverfahren.

Das DE GM 20 2013 103 766 U1 beschreibt ein Extrusionsverfahren zur Herstellung eines Schaumstoffs aus natürlichem destrukturiertem Protein wie Casein, wobei der Ausgangsstoff, vorzugsweise in Gegenwart von Plastifizierungsmitteln wie Glycerin, Zucker, Polyolen, Polyestern oder auch von physikalischen Plasitfizierungsmitteln wie Kohlendioxid, Stickstoff, Fluorkohlenwasserstoffen erhitzt und polymerisiert wird und die erhaltene teigige thermoplastische Masse durch eine oder mehrere Düsen gepresst wird. Der erhaltene Schaumstoff weist eine mechanische Stabilität auf. Welche genaue Porenverteilung oder Porengröße erhalten werden, ist nicht ausgeführt.

Auch die Gas-foaming Technologie (Zugabe von Superkritischem CO₂) findet ihren Einsatz zur Herstellung von PK, siehe US 5840,777. Dabei erfolgt die Trocknung ausschließlich durch Lyophylisierung.

Die dargestellten Techniken weisen jedoch Nachteile auf.

Eine Lyophylisierung erfolgt unter großen Zeit- und Energieaufwand, mit teuren, aufwändigen Apparaturen und diskontinuierlich betriebenen Anlagen. Auch die Reproduzierbarkeit ist sehr schwer zu steuern. Vor allem ist es sehr schwer, den für die Porengröße und Porosität verantwortlichen Eiskristallbildungsprozess zu regulieren und die PK mit gewünschter Porosität und Morphologie herzustellen.

Ohne Templating, d.h. ohne Anwendung von Porogenen (Eiskristalle, Salze, Polymere, Gase, Nanopartickel u.a.) bilden sich hingegen keine makroporösen Körper. Die herkömmliche Templating-Techniken sind noch nicht in der Lage, die hochporöse PK (> 80%) bzw. Gerüste, mit definierten Eigenschaften u.a. die mit definierten Porengröße in µm-Bereich, optimaler Porenverteilung und Porenverbindungen, mit geeigneten Morphologie, Oberflächen- und Fließ-Eigenschaften herzustellen.

Notwendige zusätzliche unvermeidliche Nacharbeitung (Verdampfung von Lösungsmittel, u.a. Wasser, oder Ätzung von Salzen, oder Nanopartikel) sind sehr aufwendig. Gas-foaming Technologien wie CO₂-Schäumungsverfahren sind durch unzureichende Reproduzierbarkeit gekennzeichnet. Ferner sind komplexe Apparaturen (Druck bis 30 Bar und höher) in diskontinuierlicher Betrieb sowie eine Lyophilisierung erforderlich. Ein kontinuierlicher Betrieb mit gleichbleibender Qualität (sog. Fließband Verfahren) kann nicht angewendet werden.

### Aufgabe der Erfindung

Aufgabe vorliegender Erfindung ist die Bereitstellung bioverträglicher hochporöser Körper (PK) mit definierter reproduzierbarer Morphologie und gewünschten physikalischen, mechanischen und biomimetischen Eigenschaften für Life Science, medizinische und/oder kosmetische Anwendungen. Dabei soll die Herstellung ohne Einsatz von herkömmlichen Porogenen und aufwendiger teurer Technik, insbesondere ohne Gefriertrocknung, Itching/ Ätzung und komprimierten CO₂ -Einsatz erfolgen.

Die PK sollen biologisch unbedenklich sein. Insbesondere soll eine effiziente Wirkstoffpenetration durch Haut und Schleimhaut, oder auch eine Anwendbarkeit als Gerüste für Zellkultivierung und Tissue Engineering, auch für Herstellung und Anwendung von Implantanten und Bioprothese möglich sein.

### Lösung

Diese Aufgabe wird erfindungsgemäß gelöst durch: Poröse Körper (PK), umfassend eine Mischung aus Polysaccharid-Naturpolymeren, ausgewählt aus Chitosan oder in einer anderen Ausführungsform aus Chitosan im Gemisch mit einem oder mehrerer Polymeren, ausgewählt aus Alginat, Alginsäure, Dextran, Xanthan, Agar, Carragenan, Carboxymethyl-, Isopropylcellulose, Pektin, Stärke, Glycosaminoglycane oder Mischungen hiervon, sowie Eiweißen, ausgewählt aus Kollagen I, II, oder III oder Mischungen hiervon, Kollagenhydrolysaten oder Gelatine, oder Mischungen hiervon, insbesondere eine Mischung aus Chitosan und Gelatine wie nachfolgend spezifiziert, mit weiterhin mindestens einer Mono/Polykarbonsäure, insbesondere ausgewählt aus Essigsäure, Propionsäure, Milchsäure, Äpfelsäure, Bernsteinsäure, Malonsäure, Fumarsäure, Ascorbinsäure, Glutaminsäure, Salicylsäure, Pyrrolidoncarbonsäure oder Mischungen hiervon, vor allem Milchsäure, Bernsteinsäure, einem oder mehreren nicht ionischen Tensiden, ausgewählt aus C₆ - C₂₂- Alkylglucosiden, C₁₀ - C₂₂- Alkylglycosiden C₆ -C₂₂-Alkyl-polyglykosiden, Sacharose- C₆ -C₂₂-Alkyl-estern, oder Mischungen hiervon, insbesondere C₆ - C₂₂ -Alkylglycosid, vor allem Decylglycosid, und vorzugsweise einem Vernetzer, wobei der

PK hexagonale oder ovale Porenstrukturen und flexible Porengrößen im Bereich von 0,1 µm bis 1000 µm, vorzugsweise 10 µm bis 700 µm, insbesondere 100 bis 650 µm aufweist. Der PK weist vor allem keine lamellaren Porenstrukturen, und/oder insbesondere keine solchen mittels Porogenen oder Porenbildnern erzeugte Strukturen, und insbesondere eine Porosität von ≥ 90% auf.

Die erfindungsgemäßen porösen Festkörper werden durch folgende Schritte erhalten:
a) Vorbereitung einer wässrigen Zusammensetzung aus Polysaccharid und Eiweiß (Bei spiel 1, 2 und 3), enthaltend Säure/n, Tensid, ggf. Wirkstoffe, und/oder Zusatzstoffe;
b) Versprühung der Zusammensetzung aus a) zu einem Schaum mit Hilfe eines Schaumsprühsystems (Beispiel 4);
c) Festkörperbildung aus dem wässrigen Schaum durch konventionelle thermische oder Strahlungstrocknung (z.B. Heißluft, Mikrowelle, Infrarot) (Beispiel 5)
d) Wahlweise Vernetzung des Festkörpers (Beispiel 6 und 7) , wahlweise Reinigung und Konditionierung (Beispiel 9), wahlweise Rehydratation zu einem 3D-Hydrogel (Beispiel 10)

Anwendungen derartiger Produkte sind in den Beispielen 13 bis 15 beschrieben.

Vorzugsweise erfolgt die Versprühung in Schritt b) durch Liquid-Gas-Filtration einer mit einem Gas, ausgewählt aus Luft, Sauerstoffangereichertem Gas mit O₂-Anteil ≥ 25%, Stickstoff, Argon, aerolisierten Zubereitung aus Schritt a).

### Abbildungen

Abb. 1 zeigt eine Vorrichtung zur Herstellung Poröser Formkörper (PK);
Abb. 2 zeigt den Einfluss der Trocknung auf den PK;
Abb. 3 zeigt die Adhäsion einer erfindungsgemäßen PK an Mucosa.
Abb. 4 zeigt die oval-hexagonale Porenstruktur eines PK, erhalten gemäß Beispiel 4.

Insbesondere betrifft die Erfindung einen porösen Körpers (PK), umfassend von 50 Gew.% bis 90 Gew.% einer Mischung aus Polysaccharid-Naturpolymeren, ausgewählt aus Chirosan, vor allem deacetyliertem Chitosan mit mittlerem MW von 70 bis 2000 KDa, vor allem 200 kDa bis 2000 kDa, sowie eines oder mehrerer Proteine (Eiweiße), ausgewählt aus Kollagen, Gelatine, Kollagenhydrolysaten, mit mittlerem MW von 20 kDa bis 60 kDa, oder Mischungen hiervon, 0,5 bis 10 Gew. % Mono- oder Bi-oder Trifunktionelle Carbonsäuren, welche eine oder mehrere Hydroxy-, Carboxy- Aminofunktionelle Gruppe oder Eis, Mischungen hiervon aufweisen, ausgewählt aus Essigsäure, Propionsäure, Milchsäure, Äpfelsäure, Bernsteinsäure, Malonsäure, Fumarsäure, Ascorbinsäure, Glutaminsäure, Salicylsäure, Pyrrolidoncarbonsäure oder Mischungen hiervon, 1,0 bis 15 Gew. % eines oder mehrerer nicht ionischer Tenside, vor allem ausgewählt aus C₆₋ C₂₂- Alkylglycosiden, C₆₋ C₂₂ Alkylpolyglykosiden, Sacharose- C₆₋ C₂₂ Alkylestern, oder Mischungen hiervon, 0 bis 20 Gew.%, bevorzugt 0,1 bis 10% wirksamen Substanzen, 0 bis 10% Hilfs- und / oder Zusatzstoffe (insgesamt 0- 30 Gew.% Zusatzstoffe, ausgewählt aus Wirkstoffen und/oder Hilfsstoffen), und 0 bis 5 Gew.%, vor allem 0,01 bis 5 Gew.% Vernetzer, 8 bis 15 Gew.% Wasser oder Restfeuchte, wobei der PK/Membran hexagonale oder ovale Porenstrukturen und eine Porengröße im flexiblen Größenbereich von 0,1 µm bis s 1000 µm, insbesondere mit wahlweise homogenem Porengrößenbereich aufweist.

Es liegt eine homogene Porenverteilung vor, wobei der Formkörper keine durch Porogene, ausgewählt aus überkritischem oder aufgelöstem CO₂, mikrokristallinen Salzen, Mikropartikeln auf Basis von Metalloxiden, Karbonaten oder Phosphaten oder Eis-Kristallen erzeugte Porenstrukturen und/oder keine lamellaren Strukturen aufweist.

Unter herkömmlichen Porogenen werden anmeldungsgemäß wie oben erläutert chemisch generiertes CO₂, z. B. erhalten durch Carbonatsalze wie Alkali-, Erdalkalicarbonate/Hydrogencarbonate, zusammen mit organischen und anorganischen Säuren wie z. B. Salzsäure oder Ascorbinsäure, oder überkritisches CO₂, oder durch Einblasen von Gas in eine Flüssigkeit/Lösung wie CO₂, N₂, Inertgas, Helium, Argon , Luft, oder nicht gasbildende Porogene, gewählt aus mikrokristallinen Salzen z.B. Alkalichloride oder Phosphate, Zuckern, Lösungsmitteln wie Glycerol, Polyethylenglykole oder Nano/Mikropartikeln wie z. B. SiO2, TiO2, Gelatinemikrosphären oder Emulsionen. Diese Porogene, insbesondere überkritisches oder gelöstes CO₂, mikrokristalline Salze, Mikropartikel auf Basis von Metalloxiden, Karbonaten oder Phosphaten werden erfindungsgemäß nicht verwendet.

Die morphologischen, physikalischen Eigenschaften, nämlich die hexagonalen oder ovalen-Strukturen der erfindungsgemäßen Membrankörper werden somit erzeugt und erhalten durch die Wahl der Komponenten der Membran, nämlich der genannten Biopolymerenmischung (Eiweiß + Polysaccharid) in den genannten Gesamtmengen von 50 bis 90 Gew.%, insbesondere 70 bis 90 , bezogen auf die Gesamtzusammensetzung, zusammen mit Tensid/en wie genannt (1 bis 15 gew.%) und Säure/n wie genannt (0,5 bis 10 Gew.%) mit wahlweise Zusatzstoffen (0 bis 30 Gew.%, vorzugsweise 1 bis 25%), Mischung der Komponenten in wässriger Lösung ohne o.g. Porogene, Verschäumen und Versprühen o.g. Zubereitung auf geeignete Flächen und insbesondere durch die konventionelle thermische und/oder Strahlungstrocknung ohne Anwendung von Gefriertrocknung und auch ohne Extrusion, da keine teigige Masse vorliegt.

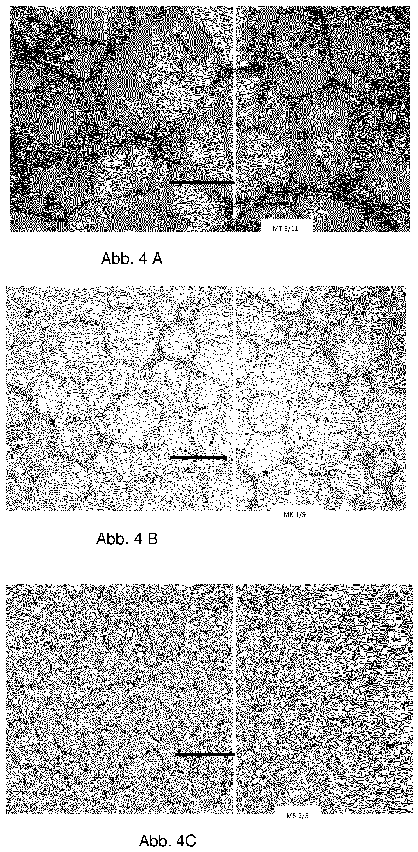

Wie in den nachfolgenden Beispielen 3, 4, 7 und Abb.4 aufgezeigt, ist der Porengrößenbereich der Membran leicht steuerbar.

Aufgrund des Fehlens der herkömmlichen Porogene, insbesondere des chemisch generierten oder überkritischen CO₂, kristallinen und amorphen Mikropartikel und/oder der Gefriertrocknung werden im Wesentlichen keine lamellaren Strukturen im Membranfestkörper erhalten.

Die erfindungsgemäße Struktur ist in Abb.4 A-C dargestellt. Abbildung 4 A zeigt die Porenverteilung einer Zubereitung, erhalten wie in Beispiel 4 beschrieben, mit GK= 1,5 %, Heißlufttrocknung, Trocknungstemperatur 55° C, 30 Minuten, mit Porendurchmesser 300 ± 100 µm, in Abbildung 4 B ist der Porendurchmesser einer Zubereitung erhalten wie in Beispiel 4 beschrieben mit GK= 2 %, Trocknungstemperatur 55° C, 35 Minuten, mit Porendurchmesser 160 ± 40 µm, in Abbildung 4 C ist der Porendurchmesser einer Zubereitung erhalten wie in Beispiel 4 beschrieben mit GK= 2 %, Trocknungstemperatur 75° C, 20 Minuten, mit Porendurchmesser 55 ± 20 µm dargestellt.

Die Poren weisen vorzugsweise eine Größe/ Durchmesser von 0,1 µm bis 100 µm, vorzugsweise 1 bis 100 µm oder auch größer 1 wie 1,2 bis 1000 µm, oder 1,5 bis 650 µm auf.

Bevorzugte strukturelle Eiweiße sind vor allem Gelatine mit einer molaren Masse von 20 kDa bis 60 kDa. Oder auch Kollagen I, II, III.

Bevorzugte Polysaccharide sind vor allem Chitosan mit einer molaren Masse von 200 bis 2000 kDA.

Insbesondere bevorzugt sind Mischungen aus Chitosan und Gelatine im Verhältnis 5:1 bis 1:5, oder auch 3:1 bis 1:1, ganz besonders 1:1.

Bevorzugte Säuren sind vor allem Essigsäure, Bernsteinsäure, Glutaminsäure, Malonsäure, Äpfelsäure, Pyrrolidoncarbonsäure, Milchsäure. Bevorzugte Mengen an Säuren sind 0,2 bis 1 Gew.%, insbesondere 0,5 bis 1,0 Gew.%.

Bevorzugte Tenside sind vor allem C₆- bis C₂₂- Alkylglucoside, insbesondere Decylglucosid. Die Erfindung betrifft auch die Herstellung derartiger PK's - insbesondere ohne Lyophilisierung - sowie deren Verwendung z. B. als 3D-Hydrogel nach Redydratation für Zellkultivierung und Tissue Ingineering, oder auch direkt als Implantat oder zur externen dermalen Applikation bei kosmetischen oder therapeutischen Anwendungen mit oder auch ohne Wirkstoff.

Erfindungsgemäße PK können hergestellt werden aus wässrigen Polymerlösungen auf Basis von Polysacchariden wie obengenannt, vor allem Chitosan und strukturellen Proteinen/ Eiweißen, insbesondere Kollagen und/oder Gelatine wie oben genannt, umfassend weiterhin nichtionische Tenside, die keine chemische Wechselwirkung mit Chitosan und Gelatine aufweisen, die genannten Säure(n), ggf. Wirkstoffe, Hilfsstoffe.

Derartige Polymerlösungen werden nach dem Zusammenmischen durch geeignete Vorrichtungen versprüht. Es entsteht ein Schaum, der sodann ohne Lyophilisierung durch konventionelle Trocknung wie thermische oder Strahlungstrocknung, Konvektion, Kontakttrocknung oder Mischungen hiervon in die gewünschte PK überführt wird. Je nach Versprühung bzw. Konzentration der Inhaltsstoffe werden unterschiedliche Porengrößen erhalten. Auch können Vernetzer bei Bedarf, z. B. zur Erhöhung einer Festigkeit, wahlweise entweder den Polymerlösungen oder im Anschluss an die Trocknung den Produkten hinzugesetzt und diese dann quervernetzt werden.

Bevorzugte strukturelle Eiweiße sind vor allem Gelatine und Gelatinehydrolysat mit einem Blomwert von 200 bis 300 oder auch Kollagen I, II, III.

Bevorzugte Polysaccharide sind vor allem Chitosan mit einer molaren Masse von 70 kDa bis 2 000 kDa.

Insbesondere bevorzugt sind Mischungen aus Chitosan und Gelatine im Verhältnis 5:1 bis 1:5, oder auch 3:1 bis 1,5:1, ganz besonders 1:1. Abhängig von dem Ziel/ der Anwendung das optimale Verhältnis Chitosan/Protein könnten jeweils passende Verhältnisse gewählt werden.,

Bevorzugte Säuren sind vor allem Essigsäure, Bernsteinsäure, Glutaminsäure, Malonsäure, Äpfelsäure, Pyrrolidoncarbonsäure, Milchsäure.

Bevorzugte Tenside sind vor allem C₁₀- bis C₂₂- Alkylglucoside, insbesondere Decylglucoside.

Derartige Zubereitungen weisen mizellenartige Chitosan-Tensid- Strukturen auf, die in den erfindungsgemäßen PK zu den differenzierbaren Zellengrößenverteilungen von 0,001 bis 2,0 mm führen.

Das Versprühen der schäumbaren Zubereitungen kann z. B. durch Aufsprühen auf Oberflächen z. B. in Gegenwart Sauerstoff, Luft oder anderer Gase zu einem stabilen, wässrigen Schaum mit definierten Zellen von 0,1 µm bis einige mm, erfolgen.

Überraschend gefunden, dass die konventionelle Trocknung, insbesondere Konvektionstrocknung, Strahlungstrocknung oder Hochfrequenztrocknung, zu einem hochporösen festen Material (PK) führt unter Erhaltung der ursprünglichen Schaum-Porosität und den gewünschten maßgeschneiderten morphologischen Eigenschaften (Porengröße, Porosität, Porenverteilung, Porenverbindungen). Eine Gefriertrocknung (Lyophilisierung) oder der Einsatz von üblichen Porogenen ist nicht erforderlich.

Geeignete Trocknungstemperaturen liegen je nach Methode im Bereich von 15 °C bis 90° C, vorzugsweise 20°C bis 75°C, insbesondere 30° bis 75°C, vor allem 35°C bis 65°C, über einen Zeitraum von ca. 60 bis 10 Minuten, je nach Temperatur.

Die erhaltenen PK weisen im Wesentlichen eine hexagonale oder ovale Porenstruktur auf, insbesondere sind sie entstanden ohne Anwendung von Porogenen, v.a. ohne chemisch generiertes oder überkritisches CO₂, kristalline und amorphe Mikropartikel und/oder umfassen keine lamellaren Strukturen.

Je nach Dicke der aufgesprühten Schicht werden entweder PK's mit einer Dicke von ≥ 1000 µm bis 2000 µm oder bis sogar einige mm oder dünnere PK's in Form von Filmen oder Membranen mit einer Dicke von , z. B. von 10 bis 1000 µm erhalten. PK/Membrane weisen, abhängig von Trocknungsverfahren und Trocknungskinetik Poren von 1 µm bis 2000 µm und eine Porosität von mehr als 90% auf. Die Innere Oberflächen-Topographie zeigt ein Netzwerk aus Kanälen und Poren im Mikrometerbereich.

Trockene PK/Membranen verfügen über eine gute Festigkeit und Elastizität.

Überraschend wurde gefunden, dass die erfindungsgemäßen PK/Membranen in der Lage sind, beachtliche Mengen von Wasser aufzunehmen (bis 10.000,0% vom eigenen Gewicht) und wandeln sich dabei in ein hochporöses 3D-Hydrogel um.

Vorzugsweise und zur Verbesserung der Eigenschaften wird die PK mittels Vernetzung bereitet. Die Vernetzung kann auf zwei Arten erfolgen, nämlich die homogene - also in der flüssigen Zubereitung/Schaum und heterogene Vernetzung - im trockenen PK- Produkt, wie nachfolgend beschrieben.

Vorzugsweise werden die getrockneten Produkte im Anschluss an die Trocknung von unerwünschten Verunreinigungen (Reste von Vernetzern, Tensiden oder Säuren) z.B. unter Anwendung von Alkoholen (Ethanol, Propanol) oder mit Wasser/Alkohol Gemische (mit Maximum 20% Wasser) gereinigt.

Trockene PK/Membranen weisen gute mechanische Eigenschaften (Festigkeit, Elastizität) auf und können maßgeschnitten oder gestanzt werden. Sie können auch in eine gewünschte Form gedreht und gefaltet werden (z.B. Röhren oder zu mehrlagigen Membranen).

### Nähere Erläuterung der Erfindung

Erfindungsgemäße PK werden bevorzugt erhalten aus folgenden Polymerlösungen, umfassend, insbesondere bestehend aus:
a) 0,1 bis 5,0 Gew. % Chitosan, vorzugsweise deacetyliertes Chitosan, 70 KDa bis 2000 kDa, vorzugsweise mit einem Deacetylierungsgrad von 60-98%, insbesondere von mehr als 70, insbesondere mehr als 80%,
b) 0,1 bis 5,0 Gew.%, Eiweiß, ausgewählt aus Kollagen, vor allem Kollagen Typ I,II und III, Gelatine,vor allem der pharmazeutischen Qualität mit Bloom-Wert von 200 bis 300, oder Mischungen hiervon,
   wobei Chitosan und Eiweiß (Kollagen und/oder Gelatine) im Mengen-Verhältnis 5:1 bis 1:5 insgesamt in einer Konzentration von 0,5 bis 10 Gew.% vorliegen;
c) 0,1 bis 5,0 Gew.% Säure, ausgewählt aus Mono- oder Bi- oder Trifunktionellen Carbonsäuren, welche eine oder mehrere Hydroxy-, Carboxy- Aminofunktionelle Gruppen oder Mischungen hiervon aufweisen, wie nachfolgend erläutert;
d) 0,1 bis 5,0 Gew. % nichtionische Tenside, ausgewählt aus C₆-C₂₂-Alkylglucosiden,C₆ - C₂₂- oder C₁₀-C₂₂- Alkylglycosiden, C₆ -C₂₂-Alkyl-polyglykosiden, Sacharose- C₆ -C₂₂-Alkylestern, oder Mischungen hiervon,
e) 0 bis 2 Gew. % Vernetzer für kovalente, chemische oder physikalische Vernetzung, ausgewählt aus bifunktionellen Aldehyden, insbesondere Glutardialdehyd (GDA), Epoxiden und Farbstoffen, insbesondere Methylenblau, Rose Bengal, Riboflavin, Curcumin;
f) 0,01 bis 5,0 Gew. % Hilfsstoffe ausgewählt aus Viskositätsregulatoren, Antioxidantien, Farbstoffen, Konservierungsmitteln, pH-Regulatoren, Lösungsmittelsvermittlern oder Mischungen hiervon;
g) 0 bis 10 Gew.%, vor allem 0,01 bis 5,0 Gew. % Wirkstoffe, ausgewählt aus kosmetischen Wirkstoffen, pharmazeutischen Wirkstoffen oder Mischungen hiervon;
h) Wasser, vor allem 60 bis 95 Gew % Wasser oder wässrige Lösung.

Basis der Ausgangszubereitung ist wie erwähnt Wasser oder wässrige Lösung aus Wasser und Alkoholen wie Ethanol und Isopropanol.

Herkömmliche Porogene sind in diesen Zubereitungen vorzugsweise nicht enthalten und werden auch nicht erzeugt oder eingeführt.

Überraschend wurde erfunden, dass die Kombination von Eiweiß/Polysaccharid, vor allem Chitosan/Gelatine(Kollagen), in bestimmten Verhältnissen mit dem/den nichtionischen Tensiden zur Bildung besonders stabiler makroporöser wässriger Schäume und aus diesen zu besonders stabilen PK's (Tabelle 7) führt. Besonders bevorzugt ist ein Verhältnis Polymer (Polysaccharid+ Gelatine) zu Tensid/en von10:1 bis 10:3.

### Erläuterung der Inhaltsstoffe

### 1.Polymere

Bevorzugt wird Chitosan (insbesondere kommerziell erhältliches mit Pharmaqualität) mit einem Molekulargewicht von etwa 70 kDa bis etwa 2 000 kDa verwendet. In einer weiteren bevorzugten Ausführungsform weist Chitosan ein Molekulargewicht zwischen etwa 100 kDa und etwa 1.000 kDa auf, vorzugsweise 400 kDa bis 900 kDa und insbesondere von 300 bis 800 kDa.

Bevorzugt wird ein deacetyliertes Chitosan, wobei der Deacetylierungsgrad von 60 bis 98%, insbesondere von 70 bis 98%, vor allem von 75 bis 98%.

Ganz besonders bevorzugt wird Chitosan mit MM von 100 kD bis 500 kDa oder vor allem 100 kDa bis 600 KDa, und einem Deacethylierungsgrad (DA) von 80% bis 95%.

Alternativ kann Chitosan teilweise ergänzt oder ersetzt sein durch andere natürliche Polysaccharide aus der Gruppe, umfassend: Alginat, Dextran, Xanthan, Alginsäure, Agar, Carragenan, Carboxymethyl-, Isopropylcellulose, Pektin, Stärke, Glycosaminoglycane aus der Gruppe umfassend Hyaluronsäure, Chondroitin, Chondroitin-4-sulfat, Chondroitin-6-sulfat, Dermatansulfat, Keratansulfat, Heparinsulfat und Heparin, oder geeignete Mischungen hiervon.

Insbesondere geeignet ist das Biopolymer Chitosan, da es im Körper mit Hilfe körpereigener Enzyme (Lysozym, N-Acetyl-D-Glucosaminidase und Chitosanase) abgebaut (biodegradiert) wird und daher eine hohe Biokompatibilität aufweist.

Chitosan kann industriell einfach aus Chitin von Crustacea, z.B. aus Krabben- oder Krill-Panzern gewonnen werden. Es wird erfindungsgemäß nicht chemisch (kovalent) durch Derivatisierung zu beispielsweise Hydroxyethyl-, Hydroxypropyl- oder Hydroxybutylchitosan verändert. Es liegt in natürlicher Form als Ausgangsmaterial vor und kann vorzugsweise durch bekannte Methoden deacetyliert werden.

Ein bevorzugtes Chitosan oder Chitosansalz z. B. Chitosan-pyrrolidoncarboxylat, welches beispielsweise unter der Bezeichnung Kytamer^{®} PC von der Firma Amerchol, USA, vertrieben wird oder Chitosan einer pharmazeutischen Qualität (wie "ChitoPharm" von Fa. Chitopharm, Norwegen). Das enthaltene Chitosan hat ein Molekulargewicht zwischen 200 kDa bis 600 kDa und ist zu bis 85% deacetyliert.

Als Protein (Eiweiß) wird vor allem Kollagen I, II, oder III oder Mischungen hiervon, Kollagenhydrolysat oder Gelatine, oder Mischungen aus Kollagen, Gelatine eingesetzt.

Alternativ kann ein Teil des gewählten Eiweißes ersetzt oder ergänzt werden durch strukturelle Eiweiße der Gruppe, umfassend Albumin, Lactoglobulin, Fibronektin und Mischungen hiervon.

Vor allem geeignet ist Gelatine, z.B. Typ A und B mit einem Bloom-Wert von 200 bis 300, z.B. MedellaPro^{®} (Gelita GmbH, Deutschland) oder Kollagenhydrolysat, MM 60 bis 70 KDa oder Mischungen hiervon mit Gelatine wie beschrieben verwendet. Alternativ oder zu-sätzlich kann auch Kollagen vom Typ I, II, III oder ein Gemisch hiervon eingesetzt werden.

Vorzugsweise ist der Polysaccharid/Protein-, vor allem Chitosan bzw. Gelatine - Lösungsprozentsatz (Gew.% / Gew.%) in der wässrigen Ausgangszubereitung größer als 0,1% Chitosan bzw. Gelatine und weniger als 5,0% Chitosan bzw. Eiweiß wie Gelatine. Mehr bevorzugt ist eine Chitosan-Gelatine-Konzentration in der Lösung von mehr als je 0,3% und weniger als je 4,5%. Am meisten bevorzugt wird die Chitosan/Eiweiß, insbesondere Chitosan/Gelatine-Konzentration in der Lösung von mehr als je 0,5% Chitosan/Gelatine und weniger als je 4 % Chitosan / Gelatine.

Verhältnisse Chitosan/ Eiweiß, vor allem Gelatine (Gew./Gew.) sind insbesondere: 20/80 bis 80/20. Am meisten bevorzugt werden Chitosan/Eiweiß, vor allem Chitosan-Gelatine - Verhältnis von 50/50 = 1:1.

Gelatine oder Kollagen wird vor allem aus Schwein- oder Rinderhaut gewonnen.

### 2. Säure

Als Säure eignen sich mono-, bi- oder trifunktionelle Carbonsäuren, welche eine oder mehrere Hydroxy-, Carboxy- Aminofunktionelle Gruppen enthalten. Diese werden insbesondere ausgewählt aus Essigsäure, Milchsäure, Äpfelsäure, Bernsteinsäure, Malonsäure, Fu-marsäure, Ascorbinsäure, Propionsäure, Glutaminsäure, Salicylsäure, Pyrrolidoncarbonsäure, vorzugsweise Essigsäure, Bernsteinsäure, Milchsäure, Ascorbinsäure, Pyrrolidoncarbonsäure, Salicylsäure oder der Kombination zweier oder mehrerer dieser Säuren.

Bevorzugt Mengen sind 0,1 bis 2 Gew.%, insbesondere 0,5 bis 1 Gew,%.

Vorzugsweise werden Milch-, Ascorbin-, Pyrrolidoncarbon- oder Bernsteinsäure oder Mischungen hiervon ausgewählt in einer Menge von mehr als 0,2% bis weniger als 4%. Stärker bevorzugt sind Bernsteinsäure und Milchsäure in einer Menge, entsprechend einem Säurelösungsprozentsatz (Gew./Gew., bezogen auf die Gesamtlösung) von mehr als 0,5% (Gew./Gew.) und weniger als 2,5%.

### 3. Tensid

Als Tenside eignen sich vor allem nicht ionische Tenside, ausgewählt aus der Gruppe, umfassend Zuckertenside wie Alkyl-Saccharoseester oder Alkyglucoside oder Alkypoly(glucoside), letztere mit 2-(Glucose-Einheiten), wobei in diesen Tensiden der Alkylteil des Esters bzw. Ethers von einer Fettsäure oder einem Fettalkohol abgeleitet ist und eine Länge von 6 bis 24, insbesondere 6-22- C-Atomen aufweisen kann. Dabei kann der Alkylteil gesättigt, einfach ungesättigt oder mehrfach ungesättigt sein wie Laurylsäure, Ölsäure, Linolsäure, Linolensäure, Myristinsäure.

Besonders bevorzugte Tenside sind ausgewählt aus C₆ -C₂₂- Alkylglycosiden, vor allem C₁₀ -C₂₂-Alkylglykosiden, C₆ -C₂₂- Alkylpolyglykosiden, Sacharose- C₆ -C₂₂- Alkylestern, C₆ - C₂₂- Fettalkoholpolyglykolethern oder Mischungen hiervon. Derartige Tenside sind im Handel beispielsweise erhältlich. Auch pflanzliche Saponine bzw. Terpene (Seifenbaum, Seifenkraut, Licorize) eignen sich.

Insbesondere bevorzugt sind C₇ -bis C₁₈ -Alkylglucoside.

Vor allem geeignet sind die folgenden Alkyl-Glucoside oder Mischungen hiervon: CAPRYLYL/CAPRYL GLUCOSIDE mit einem Alkylteil aus einer (aus Kokosöl extrahierten) sehr kurzen Fettsäure mit 6-8 Kohlenstoffatomen, im Mittel C7.

DECYL GLUCOSIDE mit (aus Kokosöl extrahiertem) Alkylteil aus Caprinsäure mit 10 Kohlenstoffatomen.

LAURYL GLUCOSIDE mit (aus Kokosöl extrahiertem) Alkylteil aus Laurylsäure mit 12 Kohlenstoffatomen. Ein Handelsprodukt hiervon ist unter dem Namen Plantacare^{®} bekannt. COCO-GLUCOSIDE mit Alkylteilen aller Fettsäuren des Kokosöls (hauptsächlich Ca-prinsäure und Laurylsäure, sowie Ölsäure, Linolsäure, Linolensäure).

Vorzugsweise werden das oder die Tenside in der Zubereitung in einer Menge / Konzentration im Bereich von 0,1 % bis 5 Gew. % (W/W), insbesondere 0,5 bis 3 Gew.%.

### 4. Wirkstoffe (pharmazeutische und kosmetische Wirkstoffe)

Erfindungsgemäß können sowohl hydrophile als auch lipophile dermato-pharmazeutische Wirkstoffe eingesetzt werden. Bevorzugt weist eine Zubereitung 0 bis 10 Gew.%, insbesondere 0,01 bis 5 Gew.% eines oder mehrerer wasserlöslicher und/oder fettlöslicher Wirkstoffe auf, ausgewählt aus der folgenden Gruppe:
Analgetika, Lokalanästhetika, Antiphlogistika,Antirheumatika, Glukokortikoide, Antibiotika, Antimykotika, Virustatika, Immunosupressiva, Immunomodu-latoren, Antipsoriatika, Keratolytika, Hormone, Phytopharmaka, pflanzliche Extrakte, Phenolcarbonsäure, Cumarine, Lignane, Flavonoide, Isoflavone, Xanthophylle, Gerbstoffe, Anthranoide , Polyphenole, Alkaloide, Glykoside, Mono- und Sesquiterpene, Triterpene, Sterole, Carotinoide, Terpenoide, Phenolsäurederivate, Vitamine und Provitamine, Antioxidantien, ätherische Öle.

Insbesondere geeignet sind Wirkstoffe aus der folgenden Liste:
- Antientzündliche Mittel (NSAID), wie Salicylate v.a. Alkyl- oder Benzylsalicylate, Indole, -Propionate, Pyrazole (Nifluminsäure ), Fenamate, Anthraniline;
- Analgetika, wie Opiate, Salicylsäure und Derivate, Gentisinsäure, Anthranilsäure und Derivate, Mefenaminsäure, Flufenaminsäure, Nicotinsäure und Derivate;
- Analgetika/Antiphlogistika, insbesondere:
   - Antirheumatika wie Heretoaryl- sowie Arylessig- und Propionsäuren-Derivate (Indometacin, Diclofenac, Sulindac), Aminobuttersäure, Thyroxin, Homocystein, Adenosylmethionin, u.a.;
- Peptide (di-, tri-, tetra-, poly-): z. B. Karnison, Glutathion, Ephitalamin u.a.;
- Vitamine und Vitamin-ähnliche Stoffe: Thiamin, Riboflavin, Niazin, Pantothensäure, Pyrridoxin, Folsäure, Kobalamin, Orotsäure, para-Aminobenzoesäure, Inosit, Carnitin, Bioflavonoide, Ascorbinsäure, Biotin, Alfa-Tocopherol, Retinol und Derivate, Beta-Karotin, Liponsäure;
- Antioxidantien (natürlichen/pflanzlichen- und synthetischen Ursprungs): Tocopherol, Carotin, Vitamin A oder Butylhydroxyanisol, Gallussäureester.
- Antivirale, fungizide und antibakterielle Mittel wie Aztreonam, Chlorhexidin-Gluconat, Amikacin, Daptomycin, Vancomycin,
- Wachstumsfaktoren oder andere biologische Stoffe, die die Wundheilung befördern.

Der oder die Wirkstoffe können bevorzugt eingesetzt werden in einer Menge von mindestens etwa 1 µg, 25 µg, 50 µg, 100 µg, 250 µg, 500 µg, 750 µg, 1 mg, 5 mg, 10 mg, 25 mg, 50 mg, 75 mg, 100 mg. Z.B. 200 mg, 250 mg, 300 mg, 400 mg oder 500 mg, wie z. B. Antibiotika.

Als pharmazeutisch-therapeutische Wirkstoffe kommen insbesondere infrage: Diclofenac, Methotrexat, Curcumin und andere Singlettsauerstoff- generierende Farbstoffe (Methylenblau, Rose Bengal oder Riboflavin).

Als kosmetische Wirkstoffe kommen vor allem Hyaluronsäure mit verschiedenen Molekulargewichten von 10 kDA bis 1000 kDA, Chondroitinsulfat mit MW von 10 kDa bis 50 kDa, Vitamine, wie Vitamin E, Vitamin C oder andere wie oben genannt, pflanzliche Extrakte (Aloe Vera, Centella asiatica und viele andere), ätherische Öle wie Lavendelöl, Rosmarinöl, Citrusöl, Orangenöl, Minzöl, Ylang Ylang, Jasminöl und viele andere, bekannte natürliche Duftstoffe eingesetzt werden.

Besonders bevorzugt werden hier als Wirkstoffe natürliche Wirkstoffe wie Polysaccharide, wie vor allem Alginate verschiedenen MW, Xanthan, Pektin, Zellulosederivate sowie Hydroxyethyl- und Hydroxypropylzellulose oder Natriumcellulosemonoglycolat, bzw. Glucosaminoglucane als Bestandteile der Extrazellulärenmatrix (ECM) Hyaluronsäure von 10 kDa bis 500 kDa, Chondroitinsulfate, Kollagen und Kollagenhydrolysate und auch Globuline (Albumin, Lactoglobulin).

Insbesondere bevorzugte Wirkstoffe sind Hyaluronsäure mit MW von 10 kDa bis 500 kDa, Chondroitinsulfat, Alginsäure und Alginate.

Die vorzugsweise vorhandenen Wirkstoffe (kosmetisch oder pharmazeutisch-therapeutisch) können erleichtert penetrieren und ihre Wirksamkeit entfalten.

### 5. Hilfsstoffe

Zu erfindungsgemäß verwendbaren Hilfsstoffen gehören u.a.:
Antioxidantien, Farbstoffe (natürliche und synthetische), Konservierungsmittel (z.B. Sensiva SC 50, Phenoxyathanol), pH-Regulatoren, Stoffe zur Beeinflussung der Viskosität und Konsistenz (Viskositätsregulatoren), Lösungsmittel wie vor allem Ethanol, Isopropanol, Propylenglycol, Glycerin oder N-Methylpyrrolidon, in einer verträglichen Menge 0,1 bis 20 %. Die Polarität von Lösungsmitteln spielt eine wesentliche Rolle bei der Freisetzung und Diffusion von Wirkstoffe.

Erfindungsgemäß umfasst die Zubereitung vorzugsweise keine lipidischen Stoffe. Hierunter werden Mono-, Di- Triglyceride von C₁₂₋₂₂-Fettsäuren oder auch natürliche Öle z. B. C₁₆-C₂₂ - gesättigte, oder ungesättigte Fettsäuren wie Olivenöl, oder Phospholipide verstanden.

Ätherische Öle (im Allgemeinen Terpene, Terpenoide, Aromaten, die keine Fette (Lipide) darstellen oder enthalten) sind erfindungsgemäß keine lipidischen Stoffe und können als Wirkstoffe enthalten sein.

### 6. Treibmittel (Gas)

Zur Versprühung bzw. Aufschäumung der wässrigen Zubereitung in geeigneten Vorrichtungen, wie in weiteren beschrieben, werden, Treibgase eingesetzt. Das Treibgas kann aber neben Luft und Sauerstoff auch andere Gase wie (Stickstoff, CO₂) enthalten bzw. dem Treibgas können zusätzlich andere Gase hinzugefügt werden. Auch Inertgas wie Argon oder Xenon sind möglich.

Der vom Treibgas erzeugte Überdruck liegt typischerweise in einem Bereich zwischen 0,3 und 4 bar, vorzugsweise zwischen 0,5 und 3 bar, besonders bevorzugt bei 0,5 bis 2 bar. Als Treibgasquelle eignen sich unterschiedliche Vorrichtungen, beispielsweise eine Gasflasche oder ein Generator. Der Schaumsprüher kann auch an eine kontinuierliche Gasleitung angeschlossen werden, so dass das System nahezu ununterbrochen betrieben werden kann. Andere Treibgase wie sonst eingesetzt wie Butan, Pentan, andere Alkane, sowie Gemische der vorstehend genannte Treibmittel sind nicht geeignet und daher erfindungsgemäß nicht verwendet.

### 7. Vernetzer

Wahlweise kann eine Vernetzung vorgenommen werden, und zwar auf zwei Arten, nämlich die homogene Vernetzung in der wässrigen Polymerlösung und die heterogene Vernetzung der festen Produkte. Die homogene Vernetzung einer Biopolymerzubereitung/ Schaum und heterogene Vernetzung kann auf zwei Arten erfolgen: zum einen kovalent und zum anderen nicht kovalent.

Eine nicht-kovalente Vernetzung (Gelierung) von Chitosan erfolgt bei der Neutralisation durch Erhöhung des pH-Wertes (pH >6,2) mit bestimmten Basen (z.B. Triethanolamin, NaH₂CO₃) oder durch lonotropische Gelierung mit Einsatz von Phosphaten oder Glycerophosphaten.

Im Gegensatz zu nicht-kovalenter Vernetzung (Gelierung) der Polymere wie Chitosan und Gelatine, erfolgt eine kovalente Vernetzung über eine chemische Reaktion. Hierzu bekannt sind vor allem (bi)funktionale Reagenzien wie Aldehyde, vor allem Glutardialdehyd (GDA), Formalin, Epoxide wie Epichlorhydrin, Carbodiimide, Bikarbonsäure (Oxylsäure, Malonsäure, Bernsteinsäure, Fumarsäure, Glutarsäure, Adipinsäure, Azelainsäure, Ascorbinsäure, Pyridincarbonsäure (PCA), Nicotinsäure, Trikarbonsäuren (Citronsäure, Tromesinsäure, Trimeeltinsäure), Aminosäure (PCA, Glutaminsäure, Arginin).

Auch Polyphenole sind als Vernetzer geeignet wie Phenolcarbonsäure (Gallussäure, Salicylsäure, Vanilinsäure), Hydroxyzymtsäuren (Kaffeinsäure, Cumarsäure, Ferulsäure), Tannine und Quinone (Hydroquinon, Benzoquinon. Ubichonon-Q-10).

Weiterhin möglich ist eine physikalische homogene und heterogene Vernetzung wie vor allem die Photochemische Vernetzung durch Umsetzung des Ausgangsmaterials mit einem photo-sensibilisierenden Reagens in einer bestimmten Konzentration. Darauffolgendes Bestrahlen der Gerüste mit einer Lichtquelle ausreichender Energie für eine bestimmte Zeitdauer führt zu Vernetzung, insbesondere zum vernetzten verschäumbaren Gel und Bildung eines PK (Xerogel) nach nachfolgendem Trocknen. Hierfür geeignete Farbstoffe sind Farbstoffe sowie Methylenblau, Rose Bengal, Riboflavin, Curcumin, u.a. verwendet (US 7393437).

Erfindungsgemäß bevorzugt sind heterogene kovalente Vernetzung mit Glutardialdehyd (GDA), Tannin sowie die physikalische Vernetzung mit Photo-sensiblen Farbstoffen, ausgewählt aus Methylenblau, Rose Bengal, Cumarin.

Auch eine enzymatische Vernetzung (Gladin, m-Transglutaminase, Laccase) von Chitosan/Kollagen bekannt.

Die Ergebnisse von Hetero- und Homo-Vernetzung sind in Tab. 5 und Tab.6 dargestellt.

### Herstellung der PK

Die verschäumbaren Ausgangszubereitungen werden hergestellt durch einfaches Mischen der Ausgangsmaterialien vorzugsweise wie in den nachfolgenden Beispielen 1 bis 3 beschrieben. Dabei kann destilliertes Wasser erwärmt werden auf z.B. mehr als 35° C bis z.B. auf 70° C. Unter Rühren werden sodann das oder die Eiweiße/Proteine (z. B. Gelatine) zugegeben und bis vollständigen Auflösung gerührt. Die erhaltene klare Lösung wird abgekühlt bis auf 25° bis 30°C. Dieser Lösung wird unter Rühren die Säure(n) zugegeben und danach das oder die Polysaccharide in der jeweils angegebenen Menge/Konzentra-tion. Die resultierende Mischung wird bis zur vollständigen Auflösung des Polysaccharides gerührt und bis auf Raumtemperatur abgekühlt. Danach wurde erfindungsgemäßen Tensid (Tenside) bei sehr vorsichtigen Rühren zugeben und bis Bildung eine klare Lösung gerührt. Sodann werden nach Bedarf API oder kosmetische Wirk- oder Hilfsstoffen (u.a. Vernetzer) hinzugefügt.

Die wässrigen (vernetzten oder nicht vernetzten) verschäumbaren Polymerzubereitungen werden sodann auf geeignete Oberflächen (Unterlagen oder Trägeroberfläche, auch Oberflächen natürlichen Ursprungs wie Haut, Wundhaut) versprüht.

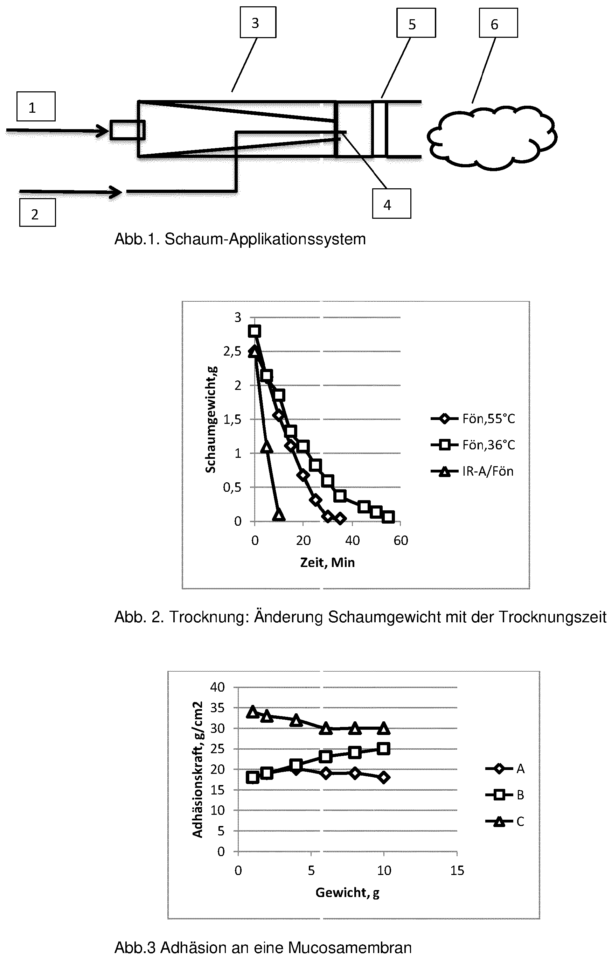

Die Verschäumung und Versprühung kann mittels einer geeigneten Applikationsvorrichtung wie z. B. in Abb. 1 dargestellt erfolgen. Hierin bedeuten: (Z) = versprühbare Zubereitung, 1: Gasquelle mit Zuführleitung und Steuerung, 2: Zuführleitung und Steuerung der Vorratszubereitung (Z), 3: Ein Halter oder Applikator (3) für die Düse (4) und für die Leitungen von Gas (1) und flüssige Zubereitung (2), 4: Venturi-Düse, 5: Schaumkopf mit Filtermaterial, 6: Schaum.

Die Applikations-Vorrichtung verfügt vor allem über eine Zweistoff-Venturidüse (4) zum Versprühen der Zubereitung (Z) mit Zuführleitung (2) sowie eine Treibgasquelle/ Treibgas-Zuführleitung (1), hier mit einer Treibgas über eine Leitung von extern. Das Treibgas treibt bei Betätigung des Applikationssystems die versprühbare Zubereitung durch die Sprühvorrichtung, vor allem Düse (4) und dadurch bildet sich ein Sprühkegel aus definierten feinen Aerosoltropfen. Es ist dabei von Vorteil, wenn die aerosolierte Zubereitung nachfolgend durch einen Filter versprüht wird. Besonders bevorzugt ist eine Kombination von Aerosolierung-Verfahren und nachfolgende Gas/Liquid-Filtration. Mit der erfindungsgemäßen Zubereitung können somit Schäume mit bedarfsdefinierten physikalischen und morphologischen Eigenschaften hergestellt werden.

Insbesondere wird die Zubereitung in einer Vorrichtung mit Venturi-Düse aerosoliert.

Venturi-Düsen sind an sich bekannt, jedoch bisher noch nicht in der Aeroliserung zusammen mit einer Filtration über einen Filterkopf zum Verschäumen einer Zubereitung eingesetzt worden.

Zerstäubungsparameter (Druck und Flow) und Filtermaterial können jeweils ausgewählt werden. Geeignete poröse Materialien für Gas/Liquid Filtration sind: Glas- und Keramikfilter, Sintermaterialien, Gewebe (z.B. Metall- oder Kunststoffgewebe), nicht gewebte Materialien (z.B. Flies). Der Schaumkopf (5) enthält vorzugsweise einen porösen bzw. aus porösem Material bestehenden Filter, z. B. Edelstahl-Siebgewebe -50 µm (SFT GmbH, Deutschland), oder eine Fritte aus Polyethylen (HDPE, Porendurchmesser ca. 20 µm (Carl Roth GmbH, DE), oder Vlies (aus Viskose, Polyester oder Gemische mit Porendurchmesser ca. 10-25 µm (POLO Filtertechnik, DE), oder Filterpapier (aus Zellulose, Porendurchmesser ca. 1-5 µm (Carl Roth GmbH, DE). Vor allem die Anwendung von nicht gewebten Materialien (Vliese) aus Polyester/Viskose und anderen Polymeren ermöglicht die Generation von geeigneten Schäumen, wie in Tab. 1 bis 3, Beispiele 1 bis 3 erläutert.

Dabei können durch geeignete Auswahl der Düsenparameter (in erster Linie BohrungsDurchmesser, Druck, Gas- und Liquid-Flow und Strahlform) sowie der Filtereigenschaften (Filterfläche, Porengröße, Porosität) aus den erfindungsgemäßen Zubereitungen stabile Schäume mit den gewünschten morphologischen/ physikalischen Eigenschaften erhalten werden.

Bei Aufschäumung der Zubereitung kann der Volumenfluss (je nach Druck) 0,1 bis 50 ml/ min bevorzugt 0,3 bis 10 ml/min und besonders bevorzugt 0,5 bis 8 ml/min betragen. Typischerweise werden auf 100 cm² Hautoberfläche von 1 bis zu 4 ml der Zubereitung verschäumt und gesprüht.

Besonders bevorzugt ist es, wenn das Treibgas und die Zubereitung Z mit einem Druck von 0,1 bis 3,0 bar und mit einer Flow-rate 0,1 bis 40 I/Min verströmt werden. Ganz besonders bevorzugt ist ein Verhältnis von Gasstrom zu Flüssigkeitsstrom (Gas-Liquid-Flow)-Verhältnis von 10 zu 1 bis 100 zu 1. Die Vorrichtung kann zwischen 0,5 bis 100 ml und mehr aufzuschäumende Zubereitung im dafür vorgesehenen Behälter aufweisen.

Aus den beschriebenen Zubereitungen werden somit vor allem dynamische Schäume mit initialem Blasendurchmesser von weniger als 0,001mm (fein-zelliger Schaum) erzeugbar. Im Laufe der Schaumreifung und je nach Zusammensetzung kann ein Schaum nach ca. 3 bis 6 Stunden (Reifung) ein (grob-zellige) Schaum mit Blasendurchmesser ≥ 1 bis mehrere mm bilden.

Die Trocknung des erhaltenen verschäumten Produktes kann auf verschiedene Weise erfolgen: thermische-, Konvektions-, oder Kontakttrocknung. Eine weitere Möglichkeit ist die Strahlungstrocknung, wobei das Trockengut von Strahlungsquellen (z. B. Infrarotstrahlern) emittierte elektromagnetische Energie absorbiert. Bei der Hochfrequenztrocknung wird das Trockengut in hochfrequenten, elektromagnetischen Feldern ausgesetzt (u.a. Mikrowellenherd). Die Aufheizung und Verdampfung erfolgt dabei nicht nur an der Oberfläche des Gutes, sondern auch in seinem Innern.

Erfindungsgemäß können alle o.g. Trocknungsarten zum Einsatz kommen. Vorzugsweise wird eine thermische Trocknung, eine Konvektions-, oder eine Strahlungstrocknung gewählt. Es können auch Kombinationen dieser einzelnen Trocknungsverfahren eingesetzt werden. Bei Aufsprühen auf biologische Oberflächen wie Haut, Wundhaut, erfolgt die thermische Trocknung sowohl durch die Oberflächentemperatur der Haut/des Gewebes wie z.B. 35 bis 37/38 °C als auch durch die Umgebungsluft wie 18°C bis 25 °C oder auch höher durch Fön etc. Hier können auch sofort mehrere Schichten übereinander gesprüht werden nach intermediärem Trocknen, wobei eine Schicht auch im Nanometerbereich wie 50 bis 500 nm sein kann. Eine derartige Sprüh-Direktanwendung (-in situ-Anwendung durch sofortige PK Bildung) ist besonders bei Hautbehandlungen von Vorteil.

Je nach Dauer und Intensität des Versprühvorganges und Trocknungsverfahrens werden PK mit einer Schichtdicke von einigen µm bis einigen mm erhalten.

Bei der Trocknung und dem Übergang vom Hydrogel zu einem Xerogel vergrößern sich einerseits die Blasen (coarsening), andererseits schrumpfen die Schaumzellen im Laufe der Wasserverdampfung. Überraschend wurde gefunden, dass durch die erfindungsgemäße Art der Trocknung ohne Lyophilisierung die Möglichkeit entsteht, Porendurchmesser und Porosität des resultierenden PK/Xerogel in breitem Spektrum einzustellen. Ferner kann mithilfe der Viskosität der Ausgangszubereitung der Blasendurchmesser und die Blasenverteilung ebenfalls beeinflusst werden. Diese kann z. B. durch die Konzentration oder MM des Polysaccharids, vor allem Chitosan wie bevorzugt beschrieben variiert werden. Mit steigender Viskosität, hier vermittelt durch die Chitosan- Konzentration, bildet sich ein Schaum mit kleinen Blasen und eine enge Blasenverteilung (Abb.2). Eine ähnliche Korrelation besteht erfindungsgemäß zwischen Schaumqualität und dem MM von Chitosan (Tab.3, Beispiel 3).

Eine Gefriertrocknung oder Lyophilisation wird erfindungsgemäß nicht gewählt.

Als Oberflächen zum Aufsprühen der versprühten schäumbaren Zubereitungen eignen sich hydrophile, hydrophobe, poröse, nicht poröse und biologische Oberflächen (Filme, Platten, Gewebe, Fliese u.a.) der Gruppe, umfassend:
- Hydrophyle: wie Zellulose, Viskose, andere Polysacchride, synthetische Polymere wie Acrylate, Polyurethane, Glas.
- Hydrophobe: Silikone, Polyurethane, Polytherephtalate, Polycarbonate, u.a.
- Poröse und Nichtporöse Materialien: Metall- und Polymerfilme, Folien aus Aluminium, Titan, Kupfer ,Polyester, Polyethylen, Polycarbonat, so wie Edelstahl Siebgewebe, Textilien, Vliese (Polypropylen, Polyester, Viskose), Verbandmaterialien (Zellulose und Mischgewebe).
- Biologische Oberflächen oder nicht biologische Oberflächen wie Dünndarmhaut, Knochen, Titan-Prothese.

Je nach Dauer und Intensität des Versprühvorganges werden die Schäume mit einer Schichtdicke von einige mm bis einige cm Dicke erhalten.

Diese werden anschließend auf einfache Weise wie bereits beschrieben getrocknet.

Die weitere optionale, insbesondere bevorzugte Aufarbeitung in Schritt d) der beschriebenen Herstellung der porösen PK umfasst ggf. Vernetzung nach Trocknung, Reinigung/ Sterilisation, Konfektionierung und Rehydratisierung oder Kombinationen dieser Schritte wie folgt:
di) Reinigung
   Das erfindungsgemäßen Reinigen von überschüssigen Resten von Vernetzer, Säuren, Farbstoffen, u.a. kann auf einfache Weise mit Lösungsmitteln wie Alkoholen, z.B. Ethanol oder Isopropanol, Polyolen (Propylenglykol), oder mit Methylpyrrolidon, u.a. erfolgen.
dii) Sterilisation Sterilisation der porösen Körper PK erfolgt durch chemische Stoffe oder durch Gammastrahlung.
diii) Konfektionierung
   Die erfindungsgemessenen PK/Membranen können in bekannter Weise z. B. durch Schneiden, Pressen oder Stanzen in verschiedene geometrische Formen (u.a. Röhren, Platten, u.a.) gebracht werden. Auch mehrere PK/Membran-Schichten können übereinander gestapelt (N-Schichten) werden. Auch können die PK/Membran zum Umwickeln von Körperbereichen wie Knochen, Zahn oder Katheter eingesetzt werden. Auch sind verschiedene Gebrauchsformen möglich: Röhren, mehrschichtige Konstrukte (Falten), Konfektionierung durch Durchlöchern, Strecken.
div) Rehydratisierung (3D-Hydrogel)

Es wurde überraschend gefunden, dass eine erfindungsgemäße getrocknete PK/Membran erhebliche Menge (bis 10.000,0% bezogen auf das Eigengewicht) an Wasser/wässrige

Lösung aufnehmen kann. Dabei wandelt sich der PK in ein hoch poröses stabiles makroporöses 3D-Hydrogel um. Sowohl der erfindungsgemäßen PK als auch die daraus nach Rehydratisierung entstandenen makroporösen 3D-Hydrogele/Scaffolds sind insbesondere geeignet für die Anwendungen in der Biomedizin wie Zellkultivierung, Tissue Engineering, Prothese, Implantate und für die andere therapeutischen/kosmetischen Anwendungen (Beispiel 13 bis 15).

### Anwendungen

Die PK ohne Wirkstoffe können aufgrund ihrer besonderen Beschaffenheit und Bioverträglichkeit für Zellkultivierung, Tissue Engineering, Prothese, Implantate, Auflagen eingesetzt werden. In Beispiel 14 ist die Züchtung von körpereigenen Fibroblasten und Stammzellen beschrieben. In ähnlicher Weise erfolgt die Kultivierung anderer körpereigener Zellen wie Nerven, Knochen oder Sehnen.

Die erfindungsgemäßen PK/Hydrogele sind je nach Vernetzungsgrad, in unterschiedlicher Geschwindigkeit in einem Körper biodegradierbar. Sie können daher je nach Anwendungsziel im Körper mit der gewünschten Kinetik abgebaut werden.

Die erfindungsgemäßen PKs weisen eine gute API- Penetration in der Haut/Schleimhaut auf und können kosmetisch und/oder therapeutisch eingesetzt werden.

Zu therapeutischen Anwendungen zählen insbesondere der Einsatz bei Dermatomykosen, Nagelentzündung, Nagelpilz, rheumatische Haut-Erkrankungen (psoriatische Arthritis), Haut-Erkrankungen durch Viren (Herpes Zoster, Pocken, Papillom, Warzen, u.a.), Ekzeme (akute-, allergische-, chronische-), Neurodermitis weiterhin auch die Geschwülste (Tumoren) der Haut, wie Aktinische Keratose, Plattenepithelkarzinom und systemische Sklerosis (Sklerodermie).

Kosmetische/dermatologische Anwendungen sind insbesondere atrophe- und hypertrophe Narben, Keloiden, Altershaut (Falten), trockene Haut, empfindliche, fleckige Haut oder bei Cellulite, Rosacea, Vitiligo, wie auch zur Hautverfeinerung und Beruhigung, oder bei Haarbehandlung wie Alopezia als auch bei der Nagelbehandlung. Zur Hautpflege, u.a. kosmetischen Indikationen können beispielsweise auch Wirkstoffe wie Hyaluronsäure, Vitamine, Antioxidantien, Peelingmittel und viele andere kosmetische Stoffe u.a. pflanzlichen Ursprungs wie vor allem Aloe Vera, Centella asiatica und viele andere pflanzliche Extrakte und ätherische Öle, Proteinhydrolysate und Peptide einzelne oder als Kombinationen mehrerer dieser Wirkstoffe eingesetzt werden. Um einen kosmetischen Effekt zu ermöglichen, sollten die erfindungsgemäßen PK/Membranen mit kosmetischen Wirkstoffen (Beispiel 2) ihre erfindungsgemäße Wirkung auf der Haut/Schleimhaut entfalten.

### Vorteile der erfindungsgemäßen PK

Die erfindungsgemäßen mikroporösen PK/Membranen können auf einfache Weise und insbesondere ohne Gefriertrocknung hergestellt werden. Durch Versprühen der Ausgangslösung - im Gegensatz zum internen Gasblasengenerieren mit Porogenmischungen (z.B. Säure + Carbonat), oder Einblasen von Gas in eine Flüssigkeit oder Lösung mit damit verbundenen Risiken (wie plötzliche Druckverringerung, Turbulenzen) - können erfindungsgemäß vermieden und damit Produkte mit jeweils reproduzierbaren morphologischen Eigenschaften wie Porengröße und Struktur erhalten werden.

Ferner sind einfache Trocknungsverfahren möglich unter Verzicht auf aufwendige Lyophiliserung.

Es wurde erfindungsgemäß gefunden, dass die PK durch Einsatz von verschiedenen Vernetzern in homo- und heterogenen Verfahren quervernetzt werden können. Insbesondere bevorzugt ist eine heterogene Vernetzung. Überraschenderweise hat sich gezeigt, dass die erfindungsgemäßen PK während ihrer Rehydratisierung erhebliche Mengen von Wasser oder Nährlösungen in sich aufnehmen können und sich in ein hochporöses 3D-Hy-drogel umwandeln. Die hervorragende morphologische und viscoelastische Gel-Eigen-schaft bietet eine Plattform für zahlreiche Anwendungen in der biomedizinischen Bereichen, u.a. Zellkultivierung/Tissue Engineering, Bioprothetik/Implantate oder Wundheilung.

Die erfindungsgemäßen PK können ferner mit darauf kultivierten eukaryontischen Zellen, unter anderen Stammzellen (Zell Konstrukte), und als nicht zytotoxische und bioabbaubare Implantate in Körperteile implantiert werden, die zu einer Regeneration des Zielgewebes führen können.

Die erfindungsgemäßen PK werden vor allem (mit oder ohne Wirkstoff) auf das zu behandelnde Körperteil oder Prothese, je nach Behandlungsziel, aufgelegt, umwickelt, oder in anderer Weise bearbeitet (z.B. Zähne, Katheder, künstliches Hüftgelenk, u.a.). Dazu kann das PK Produkt in eine geeignete Form (Festkörper Membrane oder Hydrogel) gebracht werden, welche an sich oder im Verbund mit Trägern wie Mehrschichten-PK, Folie, Transdermalen Pads, Pflastern, Prothesen usw. (siehe auch nachfolgendes Beispiele 8,12 und 13) eingesetzt werden.

Das Verfahren zur Herstellung erfindungsgemäßer PK's führt zu reproduzierbaren Produkten. Es ist daher geeignet für die Fließband-Technologie, zur Herstellung maßgeschneiderter Produkte je nach Anforderung z. B. als Auflage für kosmetisch therapeutisch Anwendung mit kleiner Porengröße oder als Gerüst oder Implantat mit höher Porengröße, wobei zahlreichen Konfektionierungen möglich sind.

Die erfindungsgemäße Herstellungstechnologie wurde für PK/Membranen entwickelt. Diese kontinuierliche Herstellungstechnologie ermöglicht, alle eingesetzten Stoffe (Lösungsmittel, Vernetzungsstoffen) umweltfreundlich zu entsorgen bzw. zu rezyklieren.

### Beispiele

Die Erfindung wird anhand der nachfolgenden Beispiele näher erläutert. In den Beispielen 1 bis 3 sind wässrige Ausgangspolymerzubereitungen mit unterschiedlichen Rezepturen dargestellt. Beispiele 4 bis 6 betreffen die Herstellung von PK (Trocknungsverfahren, Vernetzung), Beispiel 7 bis 12 Eigenschaften der PK's/ und die Beispiele 13-15 ihre Anwendungen. Die Mengenangaben sind wie oben beschrieben Gew.%, sofern nichts anderes angegeben ist.

### Beispiel 1 Schaumzubereitungen (Rezepturen 1 bis 12)

Die Rezeptur-Zubereitungen 1 bis 12 (Tab.1) werden im gleichen Modus zubereitet (hier ist beispielweiße die Rezeptur Nr.3 dargestellt): destilliertes Wasser (981,0 g) wurde in einem 1,5 I Glasgefäß unter Rühren (Magnetrührer) auf 70°C erwärmt. Danach wurden 7,5 g Gelatine (Typ MedellaPro^{®}, Gelita) zugegeben und bis zur vollständigen Auflösung gerührt. Die resultierende klare Lösung wurde bis auf 35°C abgekühlt. Zu dieser Lösung wurden unter mäßigem Rühren 4,0 g Michsäure (90%-ig) zugegeben und danach 7,5 g Chitosan-Pulver (Chitopharm). Die resultierende Lösung wurde bis zur vollständigen Auflösung von Chitosan gerührt und auf Raumtemperatur abgekühlt Zu dieser Lösung wurden 5,0 g Decylglucoside (50%-Plantacare 2001 in Wasser, BASF) zugegeben und unter langsamem Rühren bis zur vollständigen Auflösung gerührt. Es bildete sich eine klare schaumfähige Lösung. Die resultierende Lösung hatte einen pH-Wert von 5,6. Andere Tenside wurden in der Formulierung in ähnlicher Weise bei Raumtemperatur zugegeben. Das eingesetzte Chitosan (Chitopharm, Norwegien) weist ein MW von 810 kDa und eine Brookfield Viscosity von 286 cps auf. Die Gelatine hatte einen Gel Strength-Wert von 200-300 Bloom (Gelita GmbH).

Die Zusammensetzung der Zubereitungen sind in Tabelle 1 (Ingredienzien in %) zusammengestellt:

**Tabelle 1. Zusammensetzung von schäumenden Polymerlösungen**

| Zusammensetzung | 1 | 2 | 3 | 4 | 5 | 6 |
|---|---|---|---|---|---|---|
| Chitosan | 0,5 | 0,75 | 0,75 | 0,75 | 0,75 | 0,75 |
| Gelatine | 0,5 | 0,75 | 0,75 | | 0,75 | 0,75 |
| Kollagen | | | | 0,75 | | |
| Milchsäure | 0,2 | 0,4 | 0,4 | 0,4 | 0,4 | 0,5 |
| Pyrrolidonkarbonsäure | | | | | | |
| Decylglucoside | 0,1 | 0,4 | 0,5 | 0,5 | 1 | 2 |
| Sucrose Stearat | | | | | | |
| Tween-80 | | | | | | |
| Wasser | Rest | Rest | Rest | Rest | Rest | Rest |

### Fortsetzung Tabelle 1

| Zusammensetzung | 7 | 8 | 9 | 10 | 11 | 12 |
|---|---|---|---|---|---|---|
| Chitosan | 0,75 | 0,75 | 0,75 | 0,75 | 0,75 | 0,75 |
| Gelatine | 0,75 | 0,75 | 0,75 | 0,75 | 0,75 | 0,75 |
| Kollagen | | | | | | |
| Milchsäure | 0,5 | 0,5 | 0,5 | | | |
| Pyrrolidonkarbonsäure | | | | | | 0,5 |
| Decylglucoside | | | | | | |
| Sucrose Stearat | 0,5 | 1,0 | 2,0 | | | |
| Tween-80 | | | | 0,5 | 1,0 | 3,0 |
| Wasser | Rest | Rest | Rest | Rest | Rest | Rest |

### Beispiel 2: Zubereitungen mit Wirkstoffen

Ähnlich wie in Beispiel 1 beschrieben, wurden Zubereitungen mit Wirkstoffen hergestellt, wobei die Wirkstoffe/Hilfsstoffe während der Herstellung einer Zubereitung oder zu einer bereits fertigen Zubereitung zugegeben wurden, üblicherweise in Form einer wässrigen Wirkstoff-Lösung. Die Wirkstoffe wurden jeweils bei RT oder höheren Temperaturen von 30 bis 50°C, oder in passendem Lösungsmittel aufgelöst, je nach Wirkstoff- Beschaffenheit eingearbeitet. Die einzelnen Wirkstoff-Rezepturen Nr.1-10 sind in Tabelle 2 wiedergegeben. Mengenangaben in Gew.%.

**Tabelle 2. Die Wirkstoffe-, u.a. APl-erhaltene schäumenden Zusammensetzungen**

| Zusammensetzung | Rezeptur Nr. | | | | | | | | | |
|---|---|---|---|---|---|---|---|---|---|---|
| | 1 | 2 | 3 | 4 | 5 | 6 | 7 | 8 | 9 | 10 |
| Chitosan | 0,75 | 0,75 | 0,75 | 0,75 | 0,75 | 0,75 | 0,75 | 0,7 5 | 0,75 | 0,75 |
| Gelatine | 0,5 | 0,75 | 0,75 | 0,75 | 0,75 | 0,75 | 0,75 | 0,7 5 | 0,75 | 0,75 |
| Decylglucoside | 1 | 1 | 1 | 1 | 1 | 1 | 1 | 1 | 1 | 1 |
| Milchsäure | 0,2 | 0,5 | 0,5 | 0,5 | 0,5 | 0,5 | 0,5 | 0,5 | 0,5 | 0,5 |
| Ethanol | 5 | 10 | 10 | 10 | 5 | 5 | 10 | | | 10 |

| Wirkstoff | | | | | | | | | | |
|---|---|---|---|---|---|---|---|---|---|---|
| Glutaraldehyd | 0,01 | | | | | | | | | |
| Diclofenac | | 1,0 | | | | | | | | |
| Methotrexat | | | 1,0 | | | | | | | |
| Doxycyclin | | | | 0,2 | | | | | | |
| Rose Bengal | | | | | 0,01 | | | | | |
| Curcumin | | | | | | 0,01 | | | | |
| Methylen Blau | | | | | | | 0,01 | | | |
| Chlorhexidine | | | | | | | | 0,5 | | |
| nano-AgNO₃ | | | | | | | | | 0,01 | |
| Propolis | | | | | | | | | | 0.1 |
| Wasser | Rest | Rest | Rest | Rest | Rest | Rest | Rest | Rest | Rest | Rest |

### Beispiel 3 Schaumzubereitungen mit verschiedenen Chitosan MW's

Eine gewünschte Viskosität der Schaumzubereitung kann durch Variieren des Molekulargewichts (MM) und/oder der Konzentration der Biopolymere in der Formulierung erreicht werden. Wie in Beispiel 1 beschrieben, wurden folgende Zusammensetzungen hergestellt unter Verwendung von Chitosan unterschiedlicher molarer Masse wie in Tab.3 angegeben (Mengenangaben in Gew. %). Wie aus nachfolgender Tabelle 3 ersichtlich, kann ein Gel mit hoher Viskosität sowohl unter Verwendung von Materialien mit höherem Molekulargewicht als auch mit einer höheren Konzentration erreicht werden.

**Tabelle 3 Einfluss des Molekulargewichts des Chitosans und Verhältnis Chitosan/Gelatine auf die Schaumzellen Größe.**

| Inhaltsstoffe | MW von Chitosan, kDa | | | | | |
|---|---|---|---|---|---|---|
| | 310 | | | 126 | 460 | 580 |
| Chitosan | 1,1 | 0,75 | 0,4 | 0,75 | 0,75 | 0,75 |
| Gelatine | 0,4 | 0,75 | 1,1 | 0,75 | 0,75 | 0,75 |
| Milchsäure | 0,5 | 0,5 | 0,5 | 0,5 | 0,5 | 0,5 |
| Chitosan/Gelatine | 6:4 | 1:1 | 4:6 | 1:1 | 1:1 | 1:1 |
| Decylglucoside | 1,0 | 1,0 | 1,0 | 1,0 | 1,0 | 1,0 |
| Wasser | Rest | Rest | Rest | Rest | Rest | Rest |
| Schaumzellen, mm | 0,6 | 0,7 | 1,0 | 1,5 | 0,5 | 0,4 |

Das eingesetzte Chitosan war von Fa. Heppe GmbH, DE, die Gelatine Typ 200-300 Bloom von Fa. Gelita, DE und Decylglucoside von Fa. Merck, DE.

Die Schäume wurden unter Anwendung des Schaum-Applikators gemäß Abb.1 hergestellt. Aus allen Zubereitungen werden monodisperse Schäume erzeugt.

Die steigenden Molekulargewichte des Chitosans führen zu deutlicher Schaumstabilisierung, bedingt durch Viskositätserhöhung. Die Viskosität einer Zubereitung mit steigendem Molekulargewicht (MM) von Chitosan erhöht sich.

Auch das Chitosan/Gelatine-Verhältnis kann die Schaumviskosität beeinflussen. Bei wesentlich höherer Viskosität von Chitosan im Vergleich zu Gelatine führt der steigende Anteil an Chitosan zur Steigerung der Schaumviskosität und unmittelbar betroffen zur Veränderung der Schaummorphologie, ermittelt durch Schaumzellegröße (Tab.3).

Aus der Tab. 3. ist deutlich zu sehen: bei dem vorgegebenen Chitosan/Gelatine Verhältnis (1 zu 1) führt in Abhängigkeit vom steigenden Molekulargewicht des Chitosan zur Bildung (fein) kleinzelliger Schäume mit einem Durchmesser von 1,5 bis 0,4 mm. Ähnlich ist bei konstantem MM (310 kDa) die Schaumqualität in Abhängigkeit vom Verhältnis Chitosan/ Gelatine deutlich zu sehen. Eine höhere Viskosität der wässrigen Lösung kann demnach erzeugt werden durch entweder höhere Molmasse oder höheren Mengenanteil des Polysaccharids, insbesondere Chitosan, wie vor allem gleich mehr als 50% Polysaccharid, bezogen auf die Polymermischung Polysaccharid+ Eiweiß, und/oder ein MW höher 250 KDa bis 800 KDa, bevorzugt 300 bis 650 KDa Polysaccharid, vor allem Chitosan, insbesondere deacetyliertes Chitosan wie unten angegeben (Deacetylierungsgrad (DA) von 60 bis 98%, insbesondere von 70 bis 98%, vor allem von 75 bis 98%). Dies führt zur kleineren Schaumzellen und damit nach versprühen/Trocknen zu kleinen Porendurchmessern von 0,05 bis 1 mm, siehe Beispiele 3, 4 oben. Die höhere Viskosität führt zu kleineren Porendurchmessern.

### Beispiel 4 Herstellung einer PK durch Trocknung

Ein Schaum wurde aus einer Zubereitung gemäß Beispiel 1 (Tabelle 1, Nr. 3) und mit Hilfe einer Vorrichtung gemäß Abbildung 1 hergestellt. Herstellparameter: V= 2,0 ml Polymerlosung, Flow= 5l O₂/min. Der resultierende Schaum wurde in einem Kästchen/Formen aus einer glatten Polyethylen-Folie (S=30 cm², Wandhöhe-1cm) gleichmäßig aufgetragen und danach direkt mit verschieden Trocknungsverfahren (nämlich Heißluft, Mikrowelle, Infrarot) unter unterschiedlichen Bedingungen getrocknet.

Folgende Geräte wurden eingesetzt: Heizkörper (Heißluftventilator Sencor, 2 kW) mit einstellbarer Lufttemperatur (von RT bis 90 °C) und Luftgeschwindigkeit. Infrarotlampe IR-A aus Keramikstrahler, 100 W (Elstein, DE), PE- Folie Stärke 0,3mm (Folienvertrieb Drewk, DE).

Der Trocknungsverlauf wird unter konstanten Bedingungen (z.B. Trocknungstemperatur der Luft, Luftgeschwindigkeit und -feuchte) ermittelt. Die Schaumtrocknung erfolgte mit einem Abstand von Trockner und IR-Lampe von 15 cm bei unterschiedlicher Einstellung des Luftströmung Einrichtungen: von senkrecht (90 Grad) über 45 Grad, 25 Grad und waagerechte (0 Grad) Position. Es hat sich ein sehr geringerer Einfluss auf die Trocknungskinetik gezeigt. Der Trocknungsverlauf ist in der Abb. 2. dargestellt. Wie ersichtlich, korreliert die Trocknungskinetik mit den Bedingungen des Trocknungsverfahrens. Um einen Schaum zu trocknen (bis Restfeuchtigkeit ca.10%) kann wie folgt vorgegangen werden: Heißluft (36°C) - Verfahren: ca. 55 Minuten; Heißluft (55°C) ca.30 Minuten. Eine Kombination von Heißluft (55°C) und Infrarot Strahler verkürzt die Trocknung auf ca. 10 Minuten. Tempe-raturerhöhung (Heißluft 55°C) verkürzt den Trocknungsprozess um fast 30 Minuten. Eine zusätzliche Wärmequelle (IR-A- Lampe) führt zu einem noch schnelleren Trocknungsverlauf (dreifach im Vergleich zum Heißluft bei 55°C). Die Trocknungszeit spielt eine entscheidende Rolle auf Morphologie und Topographie der resultierenden PK/Membranen. Generell gilt: je schneller die Trocknungskinetik desto feinporigere PK sind herstellbar. In der Tabelle 4 sind die Ergebnisse dargestellt.

**Tabelle 4. Porendurchmesser Verteilung in Abhängigkeit von Trocknungstemperatur**

| | Zubereitung | | | |
|---|---|---|---|---|
| | Lösung 1 | Lösung 2 | Lösung 3 | Lösung 4 |
| | GK*=1% | GK=1,5% | GK=2,0% | GK=3,0% |
| Trocknungstemperatur, °C | Mittlere Zellengröße, mm | | | |
| 18,0 | > 1,0 ± 0,2 | 0,9 ± 0,2 | 0,8 ± 0,2 | 0,7 ± 0,2 |
| 36,0 | 0,8 ± 0,15 | 0,74± 0,15 | 0,62 ± 0,15 | 0,5 ± 0,2 |
| 55,0 | 0,4 ± 0,01 | 0,3 ± 0,03 | 0,3 ± 0,05 | 0, 2 ± 0,03 |
| 55,0/ bei -10°C** | 0,2 ± 0,05 | 0,1 ± 0,05 | 0,1 ± 0,05 | 0,05 ± 0,01 |

| | | | | |
|---|---|---|---|---|
| GK*: Gelkonzentration in einem Schaum, 55,0/ bei -10°C ** - Der Schaum wurde unmittelbar nach der Schaumgenerierung zuerst bei -10°C eingefroren und danach zur Trocknung bei 55°C gebracht. | | | | |

Die Porengröße wurde durch mikroskopische Aufnahme analysiert. Dafür wurde ein Digitalmikroskop (Fa. DeLi, DE) und Rasterelektronmikroskop (Fa.Zeiss) verwendet.

Es zeigt sich, dass mit der Trocknungstemperatur die Porengröße gesteuert werden kann: so können z. B. bei niedrigen Temperaturen von 18° C bis 30°C eher sehr makropöse PK/ Membranen mit Porendurchmesser von ca.1000 bis 800 µm erhalten werden, während bei höheren Temperaturen von mehr als 40° C diese sich verkleinern. Je nach angewandter Temperatur/Verfahren beträgt die Trocknungszeit im Allgemeinen bevorzugt zwischen 5 Minuten und 90 Minuten, vor allem 10 bis 60 Minuten, die Temperatur kann zwischen 15° und 80° C, bevorzugt 20° bis 60 °C liegen. Eine höhere Trocknungstemperatur führt demnach zu kleineren Porendurchmessern: Bei Temperaturen ab 60° C werden Durchmesser von 100 bis 500 µm erhalten, bei Temperaturen darunter Porendurchmesser von ca. 800 µm bis 500 µm, siehe Tabelle 4. Zwischenbereiche werden entsprechend erhalten. Bevorzugt werden Porendurchmesser von 300 bis 600 µm.

Beispielsweise weist eine Zubereitung, erhalten wie in Beispiel beschrieben erhalten aus 0,85 % Chitosan Typ Chitopharm , 0,85% Gelatine Typ Medella Pro, Gelita, 1 % Ocylglycosid, 0,5 % Milchsäure, 96,8 %Wasser nach dem Verschäumen und Versprühen mit einer Apparatur wie in Bsp. 5 beschrieben (Flow: 1,0 ml/min, Druck 0,5 bar,) und Trocknung mit einem Fön, 60° C, von 45,0 ± 6,0 µm auf, bei Zubereitungen mit je 2,5,0 % Polymer, 1% Octylglycosid, 0,7 Säure , Druck 0,7 bar und Flow 0,8 ml/min, Föntrocknung plus IR-Strahlung bei 60° C, je ca. 30 Minuten einen mittleren Porendurchmesser von 15,0 ± 3,0 µm).

Daraus und aus der Tab.4 folgt, die erfindungsgemäßen PK aufgrund ihrer morphologischen Eigenschaften als Gerüst für Zellkultivierung, Wundauflagen, Pflaster oder TD-Pflaster, Verbände, Implantate und viele andere Formen einsetzbar sind.

Ferner ist erkennbar, dass je nach Intensität der Trocknungskinetik (Heiße Luft, Wärmestrahlung oder Strahlung im Mikrowellen-Bereich) die Schaumzellen des Zubereitungsschaums erhalten bleiben können. Durch die vorhandene Blasen-/Zellenstabilität - aufgrund der Mizellen Struktur in der Ausgangszubereitung - verläuft die Schaumtrocknung ohne beeinträchtigende Kollabierung von Schaumzellen und gewährleistet die porösen Strukturen. Die Trocknung verläuft sehr schnell bis auf eine Restfeuchte von ca. 10%.

### Beispiel 5. Homogene Vernetzung eines Schaumes.

Einer Zubereitung aus Beispiel 2, Tab.2, Nr.1, wurde unmittelbar vor Schaumbildung eine 0,12% wässrige Lösung GDA-Vernetzer in verschiedenen Konzentrationen (von 0,2 bis 0,001 % zum gesamten Polymergewicht) zugegeben und 15 Minuten auf einem Magnetrührer gerührt. Das resultierende flüssige Gel wurde verschäumt und auf eine Form aus PE-Folie versprüht (so wie in Beispiel 4).Der gebildete Schaum (6x5x2 cm) wurde nach ca. 2 Minuten zur Trocknung (Heißluft/60°C, 20 Min) gebracht.

Die resultierenden Membranen wurden bzgl. der Vernetzungsgrade geprüft. Die Ergebnisse sind in Tab. 5 dargestellt.

In einer weiteren Versuchsreiche erfolgte eine homogene Vernetzung einer Zubereitung wie oben genannt mit photochemischen Farbstoffen Rose Bengal, Curcumin, Riboflavin oder Methylenblau in einer Konzentration von 0,001% bis 0,01%, in Wasser oder in Ethanol (für Curcumin). Die photochemische Vernetzung erfolgt in Anwesenheit von Sauerstoff (in die Zubereitung eingebracht z. B. mit Sauerstoffgenerator, Fa MedDrop,DE). Ein Schaum mit Vernetzer wird zu einer Lichtquelle gebracht, z. B. LED oder ein Argonlaser Ultima 2000 (Coherent Medical) mit Laserpulsen einer Dauer von 1 Sekunde bei 0,2 W. Die Gesamtenergie des Lichts, das zur Vernetzung von Chitosan/Gelatine-Gerüsten im vorliegenden Beispiel geliefert wurde, lag im Bereich von 12,5 J bis 200 J.

Nach einer vorgegebenen Bestrahlungszeit (von 10 bis 20 Min.) wurde der vernetzte Schaum zur Trocknung (siehe Beispiel 4, Heißluft 55°C plus IR-A) gebracht. Die Ergebnisse sind in Tabelle 5 zusammengefasst.

**Tab. 5 Homogene Vernetzung eines Schaumes**

| Nr. | Vernetzungsmethode | Reagenzien | Membran |
|---|---|---|---|
| chemisch | | | |
| 1 | Kovalent | GDA (Glutardialdehyd) | |
| | | 2,0 % | stark vernetzt |
| | | 1,0% | stark vernetzt |
| | | 0,5% | mäßig vernetzt |
| | | 0,1% | schwach vernetzt |

| physikalisch-chemisch | | | |
|---|---|---|---|
| 2 | Farbstoffe (protektiv) | Rose Bengal, 0,001% | mäßig vernetzt |
| | | Riboflavin, 0,001% | mäßig vernetzt |
| | | Methylenblau, 0,001% | mäßig vernetzt |
| | | Curcumin, 0,001% | mäßig vernetzt |

Die Vernetzter wurden in % zu dem gesamten Gewicht der Polymere (Chitosan/Gelatine) in die Zubereitungen gegeben.

Aus Tab.5. folgt, dass der Grad der Vernetzung in erfindungsgemäßen Zubereitungen je nach Ziel flexibel eingestellt werden kann. Die Vernetzungsbeurteilung wird in Beispiel 11 ausführlich beschrieben.

### Beispiel 6 Herstellung PK durch heterogene Vernetzung

Die heterogene Vernetzung einer getrockneten PK wurde wie folgt durchgeführt:
Eine nicht vernetzte Membrane (PK) wird aus einer Zubereitung gemäß Beispiel 1, Nr.3 durch Trocknung bei 55°C/Heißluft hergestellt und diese ca. 1 bis 5 Stunden in einer ethanolischen Lösung (25 ml) mit einem Vernetzer in vorgegebener Menge, gemäß Tabelle 6, leicht geschüttelt, danach mit Filterpapier abgetrocknet, und mit frischem Ethanol (96%) 3-mal gewaschen, abgetrocknet und anschließend bis zu einem konstanten Gewicht in der Luft getrocknet (2 bis 3 Stunden).

Das Ausmaß der Vernetzung ist in der Tabelle 6 dargestellt.

**Tab. 6. Membran-Vernetzungsmethoden (heterogene chemische Vernetzung).**

| Nr. | % Vernetzer in der Lösung | Γ_{H2O}, % | Membrane |
|---|---|---|---|
| 1 | TEA (2,0) | 8500,0±300 | schwach vernetzt |
| 2 | GDA (2,0) | 6000,0±300 | stark vernetzt |
| 3 | GDA (1,0) | 6500,0±200 | stark vernetzt |
| 4 | GDA (0,5) | 7500,0±300 | mäßig vernetzt |
| 5 | GDA (0,1) | 8000,0±150 | mäßig vernetzt |
| 6 | Tannin (2,0) | 9000,0±200 | mäßig vernetzt |
| 7 | Tannin (5,0) | 8500,0±350 | mäßig vernetzt |
| 8 | Tannin (10,0) | 8000,0±300 | stark vernetzt |
| 9 | Querzetin (3,0) | 85000,0±200 | schwach vernetzt |
| 10 | ohne | 10 000,0±300 | unvernetzt |

Es bedeuten: TEA- Triethanolamin, GDA-Glutardialdehyd.

Aus Tab.6. folgt, dass der Grad der Vernetzung in erfindungsgemäßen Zubereitungen je nach Ziel flexibel eingestellt werden kann. Es ist auch deutlich zu sehen, dass die Wasseraufnahme direkt von Vernetzungsgrad abhängig ist und mit der Erhöhung des Vernetzungsgrades sinkt.

Es ist deutlich zu sehen, dass ein heterogenes Vernetzungsverfahren in sehr einfacher und effizienter Weise eine Quervernetzung von erfindungsgemäßen PK ermöglicht.

### Beispiel 7 Vernetze PK. Morphologie

Im folgenden Beispiel wurde der Schaum (GK=1,0 Gew.%) wie in Beispiel 4 beschrieben konvektiv oder zusätzlich durch Infrarot-Trocknung getrocknet und einschließend mit 5 Gew.% Tannin und 0,5 Gew.% GDA bezogen auf die Gesamt-Polymerekonzentration heterogen vernetzt (Beispiel 6 ). Der Trocknungsverlauf wird unter konstanten Bedingungen (z.B. Trocknungstemperatur der Luft, Luftgeschwindigkeit und -feuchte, so wie in Beispiel 4 beschrieben) ermittelt.

**Tabelle 7 Morphologische und mechanische Eigenschaften von vernetzten Membranen**

| Parameter | Membrane | | | |
|---|---|---|---|---|
| | A | B | C | D |
| Stärke, µm | 98± 5 | 95±6 | 98±2 | 99±3 |
| Dichte, g/cm³ | 0,12± 0,01 | 0,10±0,01 | 0,11±0,01 | 0,10±0,01 |
| Feuchte ,% | 12,0 | 11,0 | 11,0 | 10,0 |
| Transparenz | semi | semi | semi | semi |
| Porosität, % | 96±3 | 94±4 | 94±2 | 93±3 |
| Porendurchmesser, µm | 400 ±90 | 200 ±70 | 350 ±50 | 180 ±60 |
| Porenverteilung | poly | mono | poly | mono |
| Offen-/geschlossen porig | offen | offen | offen | offen |
| Interconnection,% | 75,0±9 | 73,0±6 | 72,0±8 | 70,0±9 |
| Wasseraufnahme, % | 8500,0±300 | 8000±200 | 7500±180 | 7300±200 |
| Young's Modulus, kPa | 85±9 | 88±11 | 93±8 | 95±9 |
| Elastizität, % | 18±2 | 16±3 | 15±2 | 14±4 |

Ausgewählte PK/Membranen zur Charakterisierung:
A und C PK/Membranen (Beispiel 4, GK=1,0%) /Trocknung (Heißluft, 55°C, 25 Min),
B und D: PK/Membranen (Beispiel 4, GK=1,0%) /Trocknung (Heißluft, 55°C plus IRA-Trocknung, 10 Min)
A und C: die PK/Membranen sind mit GDA(0,5%) heterogen vernetzt (Beispiel 6, Nr.4).
B und D: die PK/Membranen sind mit Tannin (5%) heterogen vernetzt (Beispiel 6, Nr.7).

Die Morphologie erfindungsgemessener PK wurde mittels Rasterelektronenmikroskopie untersucht. Die PK wurden unter flüssigem Stickstoff in dünne Schichten geschnitten. Ihre Oberflächen wurden vor dem Scannen mit dünnem Kohlenstoff beschichtet. Tab.7 zeigt, dass die Poren im Bereich von 400µm bis 180µm (grobporöse PK) liegen und gleichmäßig verteilt sind.

Die Bestimmung der Porosität und des Anteils an geschlossenen Poren erfolgte mit Hilfe des Archimedischen Verfahrens. Dazu wurde die Trockenmasse der Membrane/PK und die Masse der PK/Membrane getränkt in DMSO bestimmt, um anhand dieser Werte die offene und geschlossene Porosität der Proben zu berechnen (Tabelle 7).

Überraschend wurde gefunden, dass in Abhängigkeit sowohl von der Trocknungsart als auch vom Vernetzungsgrad PKs mit einstellbaren morphologischen Parametern herstellbar sind.

Bei allen Proben kann eine hohe Porosität der Materialien beobachtet werden. Die Gesamt-Porosität war mit 90,0 - 96,0% sehr hoch, dementsprechend lag der reine Materialanteil bei ca. 5,5 Vol. % des Membrane-Volumens. Demnach war im Inneren der PK/ Membran ein ausreichendes Volumen für die Proliferation von Zellen und die Bildung der extrazellulären Matrix vorhanden. Zudem bestand der größte Teil aus offenen Poren und war somit von außen erreichbar, was für eine gleichmäßige Zellverteilung sowie die ungehinderte Diffusion von Nährstoffen und Stoffwechselprodukten von Bedeutung ermöglicht. Nur ca.5,0 % des gesamten PK/Membrane-Volumens waren geschlossen und demnach unzugänglich.

Das Quellverhalten der porösen PK/Membran (Tab.7, Nr. A bis D) wurde durch die Wasseraufnahme, bei 21°C in PBS (pH 7,2-7,4) ermittelt und mindestens dreimal bewertet. Eine trockene PK/Membran wurde gewogen (Wo). Nach dem Eintauchen in PBS- Lösung über 5 Minuten wurde der aufgequollene PK/Hydrogel gewogen (Wt). Das gleichgewichtige Quellverhältnis Wasseraufnahme wurde dann unter Verwendung folgender Gleichung: Γ_{H2O} (%) =( Wt- Wo)/ Wo x 100 ermittelt. Die Ergebnisse sind als Durchschnitt ± Standardabweichung dargestellt. Aus Tab.7 folgt, dass die vernetzen PK/Membranen eine hohe Wasseraufnahme aufweisen und sich in ein hochporöser 3D-Hydrogel umwandeln.

Die vernetzten PK (Nr. A bis D, Tab.7) werden einem Zug-Test unter Verwendung einer Universal Materials Testing Machine unterzogen. Die ultimative Zugfestigkeit (UTS) und der Young-Modul bestimmen die Festigkeit und Elastizität von trockenen / dehydratisierten Membranen. Für sechs Vermessungen werden die Membranen mit Längen und Breiten von 12,7 mm bzw. 3,5 mm geschnitten. Unter Verwendung einer Instron 33R, Modell 4465 (*Norwood, Mass.*) Universalprüfmaschine wurden die Zugfestigkeit (UTS) und der Young-sche Modul von PK/ Membranen vermessen.

Aus Tab.7 folgt, dass mechanische Eigenschaften der PK (Festigkeit, Elastizität) den Anforderungen an die Gerüste für Zellkultivierung und Tissue Ingineering entsprechen. Aus Tab.7 folgt weiter, dass die morphologischen und physikalischen Eigenschaften von porösen Körpern, durch Anwendung von verschieden Trocknungs- und Vernetzungsverfahren regulierbar und reproduzierbar sind.

### Beispiel 8 Trägeroberfläche

Ein Schaum wie Beispiel 1, 2 oder 3 beschrieben wurde mittels eines Schaum Applikators gemäß Abbildung 1 auf unterschiedliche Oberflächen aufgesprüht und anschließend bei Raumtemperatur ohne Vernetzung getrocknet. Die Menge betrug jeweils 1,0 ml pro 20,0 cm². Die Ergebnisse sind in nachfolgender Tabelle 10 zusammengefasst. Die Ermittlung der Adhäsion erfolgte durch Feststellung der mechanischen Abziehbarkeit des PK von der Oberfläche.

**Tabelle 10 Adhäsion von getrockneten Schäumen an nichtbiologische und biologische Oberflächen**

| Unterlage/Typ | Material-charakteristiken | Lieferant | Adhäsion |
|---|---|---|---|
| Nichtporöse, nicht biologische Oberfläche | | | |
| Glas | Borosilikat | | +++ |
| Polyethylen | LDPE, glatt, transparent, Stärke=0,3mm | (1) | + |
| Polyethylen + Vaselin | LDPE, transparent, Stärke=0,3mm | (1) | - |
| Polycarbonat | Platte glatt | (1) | ++ |
| | 24:well-Platte | (9) | ++ |
| PET | Folie, Stärke=0,2mm | (1) | ++ |
| Aluminiumfolie glatt | Stärke=0,1 mm | (2) | +++ |
| Edelstahlfolie glatt | Stärke=0,1 mm | (2) | +++ |
| Titanfolie glatt | Stärke=0,1 mm | (2) | +++ |

| Biologische Oberfläche | | | |
|---|---|---|---|
| Knoche/Rind. | | (3) | ++ |
| Schweineohr | | (3) | ++ |
| Dünndarmhaut, Kaninchen | | (3) | +++ |

| Poröse Unterlagen | | | |
|---|---|---|---|
| Siebgewebe | | | |
| Polyester | 40 Mesh | (4) | ++ |
| Polyester | 80 Mesh | (4) | ++ |
| Edelstahl Siebgewebe | 250 Mesh | (5) | +++ |

| Vlies | | | |
|---|---|---|---|
| Polyester | mittlere Durchmesser, ca. 15 µm | (6) | ++ |
| Polypropylen | mittlere Durchmesser, ca. 20 µm | (6) | ++ |
| Viskose | mittlere Durchmesser, ca. 15-20 µm | (6) | +++ |

| Filter | | | |
|---|---|---|---|
| Zellulose Filter | | (7) | +++ |
| Zellulose Michgewebe | Grob-maschig | (8) | +++ |

Die Adhäsion wurde visuell durch Abziehen der PK/Membran von der Oberfläche mit einer Pinzette überprüft: + - ganze PK war leicht abziehbar. ++ - Der PK/Membran war teilweise (fleckenweise) abziehbar, +++ - Es war nicht möglich, die Membran von der Oberfläche zu trennen. Die quantitative Adhäsion beispielsweise an der Mucoadhäsion von Membranen an Kaninchendarm durchgeführt (Beispiel 9).

Herkunft der Unterlagen: (1). Coloprint Techfilm GmbH; (2)- Metall-Ehrsberger GmbH, (3)-Schweine Ohr, Dünndarmhaut und Knochen werden aus privater Schlachterei frisch geliefert, (4)- Buckmann Co& KG, (5)- Schwegmann Filtertechnik GmbH; (6) - Polo-Filter GmbH, (7)- Schleicher & Schüll GmbH; (8) - W. Söhngen GmbH, 9 - Karl Roth GmbH, DE

Wie hieraus ersichtlich, kann je nach beabsichtigtem Ziel eine starke oder schwache Adhäsion an die Oberflächen von biologischen und nicht biologischen Unterlagen erzielt werden. Ferner zeigt sich, dass die Produkte selbst bei hoher Adhäsion stabil bleiben. Solche Produkte können ihren Einsatz in der Regenerativen Medizin, z.B. als Implantate oder Prothesen finden.

### Beispiel 9 Adhäsion der PK auf der Mucosa des Kaninchendarms

Die Adhäsion von erfindungsgemäßen PKs wurde am etablierten Model: Kaninchendarm untersucht. Eine modifizierte Balance-Methode mit zwei Armen für die Gewichte und Muster [Parodi B, et all.//Drug Deliv.Ind. Pharm. (1996);22: 445-450] wurde eingeführt, um die Bioadhäsionseigenschaften zu messen. Die Haftungstestung misst die Haftfestigkeit, d. h. die maximale Zugbelastung pro Fläche in g/cm².

Ein Kaninchendarm wurde gereinigt und in 5 cm² lange Stücke geschnittenen und mit Cyanoacrylatkleber auf eine bewegliche Plattform geklebt. Die PK/Membranen (1x1 cm =1 cm²) Nr. C (ohne Vernetzer, Tabelle 6, Nr.10), B (vernetzt mit 1,0% GDA, Tab.6, Nr.3); A (vernetzt mit 2,0% GDA,Tab.6, Nr.2), wurden auf andere Plattform mit Cyanacrylatkleber geklebt. Ein Volumen (0,1 ml) des Puffers (pH 4,8) wurde langsam unter Verwendung einer Plastikspritze über die Schleimhautprobe zugegeben. Die Plattform wurde langsam angehoben, bis die PK die Schleimhaut berührte. Die PK und die Schleimhaut wurden für 15 Minuten in Kontakt gelassen, wonach das Gleichgewicht beibehalten wurde. Die entsprechenden Gewichte (1, bis 10 g) wurden in die Pfanne des beweglichen Armes gegeben. Die Gewichtszugabe wurde nach dem Ablösen der PK/Membran von der Schleimhaut gestoppt. Die äquivalente Adhäsionskraft wurde dann in g/cm² berechnet. Jedes Adhäsionsexperiment wurde 6-mal wiederholt. Die Haftfestigkeit ist als Mittelwert dargestellt. Die Ergebnisse sind in Abb.3 dargestellt.

Aus Abb.3 ist deutlich zu sehen, dass der nicht vernetzte PK C die höchste Haftfestigkeit auf Mucosa aufweist. Der PKn A (stark vernetzt mit 2%GDA) zeigt signifikant niedrige Werte, aber ausreichende für eine gute Mucoadhäsion. Die Ergebnisse zeigten, dass die gute Bioadhäsiven von erfindungsgemäßen PK den Anforderungen an viele biomedizinische und therapeutische Anwendungen entspricht, so wie z.B. (aber nicht dadurch begrenzt) in der Drug Delivery (oral, nasal, vaginal, rektal, okular), Wundheilung, Zahnkunde, Gynäkologie oder HNO-Therapien.

Beispiel 10 Hautpenetration/ Arzneimittel Freisetzung.

### Hautpenetration von Methotrexat aus Methotrexat-haltigen PK

MTX-haltige PK (Beispiel 2) wurden für ihre potentiellen therapeutischen Einsätze bewertet. Untersucht wurden MTX-haltige PK mit unterschiedlichem Vernetzung- Ausmaß (Beispiel 2, Nr. 3 vernetzt mit 1,0 % GDA (PK A), 2,0% GDA (PK B), und 3,0% GDA (PK C).

Die Penetration von MTX in der humanen Haut (ex vitro) wurde mit Franz-Diffusionszelle untersucht.

Die Hautproben (4 × 4 cm x1, 2 mm) wurden nach Ankunft im Labor in gefrorenem Zustand (-20 °C) gelagert. Die Häute wurden in kleinere Proben geschnitten, die zur Anbringung in den Franz-Zellen geeignet waren. Die abgetaute Haut wurde mit Hilfe eines Dermatom in 500 µm Streifen geschnitten. Anschließend wurden die Hautproben über das Rezeptorkompartiment von Franz-Diffusionszellen (PermeGear, Bethlehem PA) mit einer diffusionsoffenen Oberfläche von 1,77 cm² und einem Rezeptorzellvolumen von 12 ml gelegt. Die Rezeptorlösung bestand aus PBS (pH 7,4). Die Arzneimittel- Permeation wurde 5 Stunden fortschreiten gelassen. Aufgrund der bekannten Inter-Sample-Variation in der Permeabilität von Humanhaut bestand jedes Experiment aus 6 Wiederholungen.

Die Penetration von MTX in verschiedene Hautschichten wurde mit der HPLC-Methode untersucht. Die Quantifizierung erfolgte mit der HPLC-Anlage Waters Delta (Waters GmbH, DE) mit folgenden Parametern: Saule Eurospher C-18 (Knauer, DE), mobile Phase (Acetonytril/Wasser), Flow-1 ml/min, Temperatur 40°C, UV-Detektor (304 nm). Die Kalibrierung wurde mit externen Standards erreicht. Die Probenretentionszeit betrug 9 Minuten und die Quantifizierungsgrenze des Verfahrens betrug 0,1 µg/ml. Jeder gemessene Wert in µg/cm² repräsentiert den Mittelwert und Standardfehler des Mittelwerts.

Die Ergebnisse ex vivo Penetration von MTX wurden in der Tab. 9 dargestellt.

**Tabelle 9. Penetration in der Haut in % der applizierten Dosis von MTX (n=6) aus MTXhaltigen Membranen**

| Membrane | Hautschicht | | | Akzeptor | Penetrierter Anteil, % |
|---|---|---|---|---|---|
| | SC | EPI | DER | | |
| A | 3,45±1,22 | 6,12±1,52 | 5,80±1,42 | 2,61±0,20 | 15,5 |
| B | 2,26±0,87 | 4,57 ±0,76 | 3,31±1,13 | 2,38±0,82 | 9,8 |
| C | 1,58±0,76 | 2,52±0,84 | 1,24±0,98 | 2,12±0,45 | 5,3 |

### CS- Stratum corneum, EPI- vital Epidermis, DER- Dermis

MTX konnte aus allen PK/Membranen in Haut- Kompartimente penetrieren. In der lebenden Epidermis konnten für die PK A und B ca. 4,5 bis 6 % in der Epidermis und ca.3, 3 bis 5,8% in der Dermis des applizierten MTX quantifiziert werden. Die Abhängigkeit der Penetration vom Vernetzungsgrad der PK war statistisch relevant: Je höher der Vernetzungsgrad, desto langsamer penetriert MTX in die Hautkompartimente. Für den PK C wurde eine langsame Abgabe von MTX festgestellt. MTX konnte nach Applikation danach nur in geringer Konzentration im Akzeptorkompartiment nachgewiesen werden.

Die wirkstoffhaltigen PKs insbesondere mit MTX, können daher beispielsweise zur lokalen topischen Anwendung als aktives Element in einen transdermalen Patch/Pflaster z.B. für die Therapie von Gelenk-Entzündungen und -Schmerzen, so wie Psoriasis oder aktinische Keratose eingesetzt werden.

### Beispiel 11: Antimikrobielle Wirkung

Zu den Untersuchungen wurden für eine chronische Wunde herkömmliche Bakterien S. aureus 209, P. aeruginosa, E. coli und Hefen Candida albicans genommen.

Aus einem flüssigem Nährmedium (Standard Nährmedium Boulion, VWG-Euro-Lab, Darmstadt) mit einer darin zur stationären Phase gewachsenen Kultur wurde eine 1: 200- Verdünnung bis zum Erreichen einer optischen Dichte bei eine Wellenlänge von λ =550 nm (OD₅₅₀) von 0,125±0,02 hergestellt. Diese Trübung entsprach der Konzentration N=10⁸ KBE/ml. Ausgehend von dieser Suspension wurden Aliquots entnommen und in frische Bouillon gegeben, so dass die gewünschte Koloniezahl von10², 10⁴ und 10⁶ KBE/ml resultierte. Dazu wurden gleich große Stücke (ca. 2x2 cm) von PK's/Membranen mit Chlorhexidin, mit Nano- Ag-Partikel und Kontroll-PK's ohne Wirkstoffe (Beispiel 3, Nr. 1, 8 und 9) gegeben und in eine Petrischale insgesamt bis 48 Stunden inkubiert.

Die bakteriostatische Aktivität der Schwämme wurde bestimmt, indem die Testkulturen direkt dem Nähragar in einer Menge von 10²-10⁶ KBE / ml zugegeben wurden. Nach 48 Stunden Inkubation wurde der Durchmesser der Verzögerungszone des Mikroben-Wachstum um die PK herum gemessen. Die Ergebnisse sind in Tab.10 zusammengefasst.

**Tabelle 10. Kolonien Anzahl Veränderung von S. aureus 209 ST547, P. aeruginosa TCC27583A , E. coli ATCC25922 und Hefen Candida albicans in Nährbouillon mit verschiedene Membrane.**

| Testorganismen | Impfung (KBE/ml) | KBE nach 48 Stunden | | |
|---|---|---|---|---|
| | | PK/Membran mit Chlorhexidin (Nr.8) | Membrane mit Ag, (Nr.9,) | Membrane (Nr.1) |
| S. aureus 209 | 1.7×10² | 2 ±0.2 | 0 | 0 |
| | 1.7×10⁴ | 44 ±5 | 13 ±2 | 50 ±1 |
| | 1.7×10⁶ | 54 ±6 | 30 ±6 | 150 ±6 |
| E. coli | 2.5×10² | 0 | 0 | 0 |
| | 2.5×10⁴ | 0 | 0 | 20 ±6 |
| | 2.5×10⁶ | 50 ±6 | 25 ±6 | 110 ±10 |
| P. aeruginosa | 1.9×10² | 0 | 30 ±4 | 50 ±4 |
| | 1.9×10⁴ | 0 | 60 ±8 | 98 ±1 |
| Candida albicans | 1.4×10² | 0 | 0 | 0 |
| | 1.4×10⁴ | 0 | 0 | 5 ±1 |

Die PK/Membranen sind aus Beispiel 2.

Wie ersichtlich, kann mit Chlorhexidin Bigluconat, bzw. mit Ag⁺- Ionen versehener PK/Membranen gemäß vorliegender Erfindung, das Wachstum von P. aeruginosa und C. albicans auf Agar vollständig und fast vollständig das Wachstum von S.aurens und E. coli. gehemmt werden. Der Ausgangs PK ohne Wirkstoff (Nr.4, Tab.6 ) hemmt auch vollständig das Wachstum von C. albicans und fast vollständig das Wachstum S.aurens und E. coli in Konzentrationen bis 2.5×10⁴ KBE / ml. Das Wachstum von S. aurens und E. coli in Konzentrationen über 10⁶ KBE / ml war ebenfalls inhibiert. Da die Anzahl an Krankheitserregern (z. B. in eitrigen Wunden) selten mehr als 10⁴ KBE/ml in einer Wundflüssigkeit erreichen kann, ist ersichtlich, dass die entwickelten wirkstoffhaltigen PK (Membrane) eine ausgeprägte antimikrobielle Wirkung aufweisen. Auch die PK ohne Wirkstoffe zeigen eine gute bakteriostatische Wirkung.

### Beispiel 12 Degradation in vitro

Die Stabilität der PK in biologischen Lösungen ist für ihre Anwendungen im Bereich Zellkultivierung, Gewebszüchtung und Bioprothetisierung von entscheidender Bedeutung. In diesem Beispiel wurde die Stabilität von erfindungsgemäßen PK(Membranen)/Gele in vitro in einem Phosphatpuffer und in einem Enzymhaltigem Lösungen dargestellt. Die Degradationskinetik wurde hier auch als ein Kriterium für den Vernetzungsgrad erfasst. Die Degradation wurde durch die Stabilität der vernetzten PK in einer sterilen Phosphat- Puffer-Lösung (pH =5,5) im Laufe von bis 3 Monaten bei Raumtemperatur geprüft. Nach 3, 15, 30 und 90 Tagen blieben die PK stabil (unverändert).

### Enzymatische Degradation.

Die Degradation von vernetzten PK wurde durch Inkubation mit Kollagenase (EC3.4.24.3 Clostridium histoliticum, mit einer spezifischen Aktivität von 414 U/mg, Sigma) überprüft. Hierfür wurden 100 mg PK/Membrane in Röhrchen gelegt, 0,5 ml Tris HCl Lösung (pH 7,4) und zusätzlich 0,0,5 g Natriumazid hinzugefügt. Nach 0,5 Stunden Inkubation bei RT wurden 0,5 ml Kollagenaselösung in Tris-Puffer hinzugegeben, bis zur Konzentration in der Inkubationslösung von 100g/ml, d.h. 10U/mg PK. Nach 1,0, 6,0; 18 und 24 Stunden bei RT wurden Proben entnommen, mit destilliertem Wasser gewaschen und gefriergetrocknet. Die Degradation wurde in Gewichtsverlust (in %) zu dem Initialen Gewicht ermittelt. Die Ergebnisse sind in Tab.11 aufgezeigt.

**Tab.11. Dergradationskinetik vom vernetzte PK/Gel in Kollagenase- Lösung.**

| Vernetzer | Gewichtsverlust (%) nach | | | |
|---|---|---|---|---|
| | 1 Stunde | 6 Stunden | 18 Stunden | 24 Stunden |
| GDA (2,0%) | - | 10±6 | 46±5 | 99±7 |
| GDA (1,0 %) | - | 34±6 | 78±7 | 99±8 |
| GDA (0,1%) | 13±5 | 67±4 | 98±7 | - |
| Tannin (2%) | 21±3 | 78±5 | 98±6 | - |
| Tannin (5%) | - | 26±4- | 55±6 | 98±3 |
| Tannin (10%) | - | 13±5 | 46±1 | 98±2 |

### GDA: Glutaraldehyd.

Aus Tab.11 es ist deutlich zu sehen, dass stark mit GDA (1,0 und 2,0 %) oder Tannin (5,0 und 10%) vernetzte PK in der Enzym-Lösung nnerhalb 24 Stunden biodegradiert werden. Schwach vernetzte PK/Membrane (GDA 0,1% und Tannin 2%) sidn bereits nach 6 Stunden wesentlich degradiert. Die enzymatische Abbaubarkeit von erfindungsgemäßen PK ist eine gute Voraussetzung für eine Biodegradation/Bioabbau in einem Körper.

### Beispiel 13 Hydrogele

Überraschend wurde erfunden, dass die PK nach ihrer Anfeuchtung, in ein stabiles 3D-Hydrogel umgewandelt werden können, was für weitere zahlreiche medizinisch-biologi-schen Anwendungen vorteilhaft ist.

Für Rehydratisierung wurde der PK/Membrane auf 2x 2 cm = 2 cm² geschnitten. Das Quellverhalten des porösen PK (Tab.7, Nr. A bis D) wurde bei 21°C in PBS (pH 7,2-7,4), wie in Beispiel 7 beschrieben mindestens dreimal bewertet.

Die erfindungsgemäßen vernetzen PK/Membranen zeigten je nach Vernetzungsgrad eine hohe Wasseraufnahmekapazität, z. B. bis 100 Mal, bezogen auf das eigene Gewicht für gering vernetzte PK/Membrane. Die erfindungsgemäßen stabilen Hydrogele mit ihren hervorragenden morphologischen und mechanischen Charakteristiken sind geeignet für ihre Anwendungen in den Bereichen Zellkultivierung, Tissue Engineering oder Bio-Implantation geeignet.

### Beispiel 14 In vitro Zellkultivierung von Fibroblasten (hFb) und Stammzellen (hMSC).

Für die Kultivierung der oben erwähnten Zellen wurden die PK/Membran A (Beispiel 6, Nr.8), PK B (Beispiel 6, Nr.3) und PK/Membran C (Beispiel 6, Nr.2) mit unterschiedlichen morphologischen Charakteristiken (Vernetzungsgrad, Porengröße und Porosität) verwendet. Für die Kultivierung wurden die PK (Dicke 100µm) mit Durchmessern von 8 mm, passend zu einer 48-Well-Kultivierungsplatte, gestanzt und erfindungsgemäß mit Ethanol sterilisiert. Zur Kultivierung wurden humane dermale Fibroblasten (hFb) von einer Hautbiopsie gezüchtet. Zelllinien wurden in Dulbecco's Modified Eagle's Medium/High Modified (DMDE), Nährlösung, ergänzt mit 10% fötalem Rinderserum (Gibco TM) und 1% Antibiotikum / Antimykotikum (Gibco TM) in einem angefeuchteten CO₂ -Inkubator bei 37 ° C mit mittlerer Erfrischung jeden zweiten Tag kultiviert.

Für die Kultivierung wurden hFb-Zellen (5,0 × 10⁵ Zellen/ml) in DMDE-Nährlösung suspendiert bis zu einer Konzentration (5,0 × 10³ Zellen/ml). 100 µl der Zellimpflösung wurden auf jedem PK in 48 Well-Platten aufgetragen und nach 10 Minuten Inkubation bei 37°C 1,0 ml DMEM zugegeben. Danach wurden die Zellen für 1 h weiter inkubiert. Die genaue Zelldichte in jeder Lösung wurde bestimmt mit Hilfe von automatisierten Sceptre^{™} 2.0 Zellzähler (Merk, DE). Die hFb Zellen wurden bis zu ihrer Differenzierung kultiviert (ca. 3W).

In ähnlicher Weise wurden Mesenchymal Stem Cells (hMSC) kultiviert. 4,5 10⁵ mesenchymalen Stammzellen (Lonza, Basel) wurden auf die Gerüste aufgetragen. Als nächstes wurde 1 ml Clonetics^{™}/Poietics^{™} Nählösung zu jeder Kultur zugegeben. Die Zellen wurden für drei Wochen kultiviert und die Nährlösung alle 48 Stunden gewechselt.

Es wurden zwei Impfmethoden verglichen: sog "Immobilisierungsmethode" (IM) und "Inokulierungsmethode" (OM). Bei der IM-Methode wurde die hFb (oder hMSC) in eine Konzentration von 5x 10³ Zellen/ml zu der trocknen PK/Membran (in eine 48-Well Kulturplatte) zugegeben. Nach Rehydratation der PK/Membrane unter Bildung eines Hydrogels wurden 1,0 ml DMDE vorsichtig oberhalb des Hydrogels einpipettiert. Die Zellen werden in einem Inkubationsschrank innerhalb von 3 Wochen kultiviert.

Bei der OM-Methode wurde der PKin 48-Well-Platten in der Nährlösung rehydratisiert unter Bildung eines Hydrogels. Anschließend wurde dieses mit 5x 10³ hFb -Zellen/ml geimpft und in einem Inkubationsschrank innerhalb von 3 Wochen kultiviert.

Die Zytokompatibilität der getesteten PK(Membran)/Hydrogele zu den hFb-und hMSC-Zelllinie wurde durch Bestimmung von zell-biologischen Parametern wie Zell-Adhäsion, Proliferation und Migration untersucht. Die Effizienz von Zellkultivierung auf untersuchten PK/Hydrogelen wurde durch Effizienz der Zelleneinsiedlung (Seeding efficiency), Zellenverteilung in der Matrix (Cell distribution), Zellen Viabilität (Cell viability) und Zellen Proliferation untersucht.

Der Nachweis für die Eignung von erfindungsgemäßen Hydrogelen für Tissue-Engineering wurde unter Verwendung verschiedener analytischer Techniken, einschließlich Lebend/ Tot-Färbung, Fluoreszenskolorimetrie, Konfokal-Laser-Mikroskopie (CLSM) und histologischer Untersuchung an den mit Zellen bewachsene Hydrogelen bewertet. Diese sind bekannt und beschrieben in: Berridge, M. Vet al. The Biochemical and Cellular Basis of Cell Proliferation Assays That Use Tetrazolium Salts. In: Biochemica (1996); Chan LL, et al. Rapid image-based cytometry for comparison of fluorescent viability staining methods. // J Fluoresc. 2012; 22(5):1301-11] [Cell staining // in www.dojindo.com/Protocol/CellStaining_ Protocol.pdf]; [Martha O., et al. Evaluation of the In Vitro Cytotoxicity of Crosslinked Biomaterials//Bioma-cromolecules. 2013 May 13; 14(5): 1321-1329]; [James B. Pawley (Hrsg.): Handbook of biological confocal microscopy. 3rd Edition. Springer Science and Business Media - LLC, New York NY 2006], sowie in Protokolls und Bedienungsanleitungen von Geräten und Reagenzien.

### Ergebnisse:

Zuerst wurde festgestellt, dass aus PK/Membranen entstandene Hydrogele gegenüber beiden Inkubationsmedien im Laufe von 30 Tagen inert und nicht zytotoxisch waren (Beispiel "Hydrodel"). Die Hydrogele sind haltbar sowohl in Nählösungen als auch in Ringer-Flüssigkeit und reagieren nicht mit ihren Zutaten. Daher kann die Möglichkeit der Bildung toxischer Abbauprodukte oder Produkte chemischer Reaktionen mit Inkubationslösungen eliminiert werden.

Die dreiwöchigen Zell Züchtungen von Fibroblasten und hMSC wurden an den vernetzten mit GDA PK/Membranen (Beispiel 6, Tab.6, Nr.2, 3 und 4) und nicht vernetzten PK/ Membran-K (Beispiele 1, Nr.5) in Abhängigkeit von der Kultivierungsmethode unter Anwendung obengenannter analytischer Verfahren untersucht. Die Ergebnisse sind in Tab.12A und Tab. 12B zusammengefasst.

**Tab. 12A. Kultivierung von Fibroblasten**

| Kultivie rung | "Immobilisation"- Methode | | | | "Inokulation"- Methode | | | |
|---|---|---|---|---|---|---|---|---|
| | GDA, % | | | | GDA, % | | | |
| | K (ohne GDA) | 0,5 | 1,0 | 2,0 | K(ohne GDA) | 0,5 | 1,0 | 2,0 |
| | Besiedelung mit Zellen / Penetration in Gerüst | | | | | | | |
| 24 h | 90,0±5 | 90,5±6 | 85,6±8 | 80,5±6 | 80,2±9 | 79,4±6 | 67,7±7 | 58,5 ±9 |

| | Zell Viabilität,%, | | | | | | | |
|---|---|---|---|---|---|---|---|---|
| Am 4 Tag | 98,3±2 | 96,6±3 | 97,0±2 | 98±1 | 98±2 | 97±1 | 98±1 | 97±2 |

| | Zellen Proliferation | | | | | | | |
|---|---|---|---|---|---|---|---|---|
| 4 Tagen | 10⁴ | 10⁵ | 10⁵ | 10⁵ | 10⁴ | 10⁴ | 10⁴ | 10⁴ |
| 12 Tagen | 10⁵ | 10⁶ | 10⁶ | 10⁶ | 10⁶ | 10⁶ | 10⁶ | 10⁶ |
| 21 Tag | 10⁶ | 10⁶ | 10⁶ | 10⁶ | 10⁶ | 10⁶ | 10⁶ | 10⁶ |

**Tab. 12B. Kultivierung von Stammzellen (hMSC)**

| Kultivierung | "Immobilisation"- Methode | | | | "Inokulation"- Methode | | | |
|---|---|---|---|---|---|---|---|---|
| | GDA, % | | | | GDA, % | | | |
| | K (ohne GDA) | 0,5 | 1,0 | 2,0 | K (ohne GDA) | 0,5 | 1,0 | 2,0 |
| | Besiedelung mit Zellen / Penetration in Gerüst, % | | | | | | | |
| 24 h | 88,0±5 | 86,5±6 | 87,6±8 | 80,5±6 | 80,2±9 | 77,4±6 | 68,5±7 | 60,5±9 |
| 48 h? | 90,0±5 | 91,5±6 | 92,6±8 | 82,5±6 | 83,2±8 | 80,4±6 | 79,7±7 | 78,5±9 |

| | Zell Viabilität,%, | | | | | | | |
|---|---|---|---|---|---|---|---|---|
| Am 4 Tag | ca.90,0 ±5 | ca.90,0 ±5 | ca.90,0 ±5 | ca.90,0 ±5 | ca.90,0 ±5 | ca.90,0 ±5 | ca.90,0 ±5 | ca.90,0 ±5 |

| | Zell Proliferation | | | | | | | |
|---|---|---|---|---|---|---|---|---|
| Nach 6 Tage | 10⁴ | 10⁵ | 10⁵ | 10⁵ | 10⁴ | 10⁴ | 10 ⁴ | 10⁴ |
| Nach 12 Tage | 10⁵ | 10⁶ | 10⁶ | 10⁶ | 10⁶ | 10⁶ | 10⁶ | 10⁶ |
| Nach 21 Tage | 10⁶ | 10⁶ | 10⁶ | 10⁶ | 10⁶ | 10⁶ | 10⁶ | 10⁶ |

Die Ergebnisse von In-vitro-Studien zeigen, dass Hydrogele aus erfindungsgemäßen porösen PK/Membranen sehr gute Zytokompatibilität zeigen und dadurch geeignete Kandidaten für die Zellzüchtung sind.

Beispiel 15 Beschleunigung der Wundheilung mit Hilfe einer PK ( Membrane) und Stammzellen-Konstrukt.

Überraschend wurde erfunden, dass erfindungsgemäße PK/Membrane Wundheilungsprozesse beschleunigen. Die Aktivierung und Proliferation von Fibroblasten, Kollagensynthese, Neo-Angiogenese und stimulierende Migration von Zellen im Wund Bett sind für den Wundheilungsprozess von entscheidender Bedeutung. In dieser Studie wurde die Wirkung einer hMSC-besiedeltem PK/Membran (aus Beispiel 14) im Vergleich zum unbesiedelten PK untersucht.

Die Studie wurde an 60 weißen männlichen geschlechtsreifen Wistar Ratten mit einem Körpergewicht von 180-230 g durchgeführt. Die Tiere wurden in drei Gruppen randomisiert, davon wurden 20 Tiere wurden mit Membrane (Beispiel 6, Tab.6, Nr.8), 20 Tiere mit hMSC-Konstrukt (Beispiel 14) behandelt. 20 Tiere aus der Vergleichsgruppe wurden mit 0,9% iger isotonischer NaCl-Lösung behandelt. Das Modell einer vollschichtigen Wunde wurde unter Anästhesie im Rückenbereich, durch Exzision des Hautlappens mit subkutanem Faszien in Form eines Quadrats in der Größe von 20 × 20 mm gebildet. Die Wundheilung wurde am 7., 14. und 21. postoperativen Tag beobachtet.

Die erzeugten Wunden an den Versuchstieren wurden an den postoperativen Tagen 0 bis 21 unter Verwendung einer hochauflösenden Canon-Digitalkamera (PowerShot G7; Canon,) fotografiert. Die Fotografien wurden mit dem Softwarepaket analysiert. Der Prozentsatz des Wundverschlusses (Kontraktion) wurde wie folgt definiert: (Wundfläche am postoperativen Tagₓ) / (Wundfläche am postoperativen Tag₀) × 100. Der Verlauf des Wundheilungsprozesses ist in Tabelle 13 zusammengefasst.

**Tabelle13. Wundverschluss bei Ratten im Laufe des Wundheilungsprozesses**

| Tag | K | Membran | hMSC-Konstrukt |
|---|---|---|---|
| 4 | 46±15 | 58±15 | 67±18 |
| 7 | 55±17 | 75±13 | 89±11 |
| 14 | 76±10 | 91±8 | 100±2 |
| 21 | 84±10 | 100±5 | |

Die planimetrischen Daten zeigen, dass sowohl mit der erfindungsgemäßen PK/Membran als auch vor allem mit dem daraus erhaltenen hMSC-Konstrukt gegenüber der Kontrollgruppe ein verbesserter Wundverschluss erfolgte, wie ersichtlich festgestellt am 4., 7., und 21. postoperativen Tag. Die vollständige Epithelisierung war in der hMSC-Konstrukt-Gruppe ca. eine Woche früher im Vergleich mit der PK-Gruppe und ca. 2. Wochen früher als in der Kontrollgruppe. Die quantitative Analyse des hMSC-Konstrukts gegenüber der PK/Membrane zeigte eine Verstärkung der Neoangiogenese. Die Mikrogefäßdichte bei zwei Wochen nach der Operation entnommenen Wundbettbiopsien war signifikant höher in der hMSC-Konstrukt-Gruppe im Vergleich zum PK alleine (7,5 ± 1,1 vs. 5,1 ± 1,0 Gefäße pro Hochleistungsfeld) und Kapillardichte: - (800 /mm² vs.420/mm²)..

Die Studienergebnisse haben deutlich gezeigt, dass menschliche Stammzellen (Konstrukt aus Beispiel 14), die auf einem Chitosan/Gelatine-Gerüst ausgesät werden, die Wundheilung beschleunigen und eine Differenzierung in fibrovaskuläre-, endotheliale- und epitheliale- Komponenten im wieder hergestelltem Gewebe zeigen.

## Patentansprüche

1. Poröser Körper (PK), umfassend 50 Gew.% bis 90 Gew.% einer Mischung aus Polysaccharid-Naturpolymeren, ausgewählt aus Chitosan, deacetyliertem Chitosan mit mittlerem MW von 70 kDa bis 2000 kDa, vor allem 200 kDa bis 2000 kDa, sowie Eiweißen, ausgewählt aus Kollagen I, II, oder III oder Mischungen hiervon, Kollagenhydrolysat oder Gelatine mit mittlerem MW von 20 kDa bis 60 kDa, oder Mischungen hiervon, mit weiterhin 0,5 bis 10 Gew.% mindestens einer Mono-/Polycarbonsäure, ausgewählt aus Essigsäure, Propionsäure, Milchsäure, Apfelsäure, Bernsteinsäure, Malonsäure, Fumarsäure, Ascorbinsäure, Glutaminsäure, Salicylsäure, Pyrrolidoncarbonsäure oder Mischungen hiervon, sowie 1,0 bis 15 Gew. % von einem oder mehreren nichtionischen Tensiden, ausgewählt aus C₆ - C₂₂ - Alkylglucosiden,
C₁₀-C₂₂-Alkylglycosiden, C₆-C₂₂-Alkyl-polyglykosiden, Saccharose- C₆ -C₂₂-Alkylestern,
0 bis 30 Gew. % Zusatzstoffen, wahlweise ausgewählt aus Hilfs-und Wirkstoffen, 8 bis 15 Gew.% Wasser oder Restfeuchte, wobei der hochporöse Körper hexagonale oder ovale Porenstrukturen, sowie flexibel einstellbare Porengrößen im Gesamtbereich von 0,1 µm bis 1000 µm, vorzugsweise 10 µm bis 700 µm, mit homogener Porenverteilung und keine durch Porogene, ausgewählt aus überkritischem oder aufgelöstem CO₂, mikrokristallinen Salzen, Mikropartikeln auf Basis von Metalloxiden, Karbonaten oder Phosphaten oder Eis-Kristallen erzeugte Porenstrukturen und/oder keine lamellaren Strukturen aufweist.

2. PK nach Anspruch 1, **dadurch gekennzeichnet, dass** als Zusatzstoffe, pharmazeutische und/oder kosmetische Wirk- und Hilfsstoffe vorhanden sind.

3. PK nach einem der Ansprüche 1 oder 2, **dadurch gekennzeichnet, dass** der PK vernetzt ist mit einem Vernetzer, ausgewählt aus bifuktionellen kovalenten chemischen Vernetzern, Glutardialdehyd, Carbodiimid oder aus photochemischen Vernetzern Methylenblau, Cumarin, Riboflavin oder Bengal Rose.

4. PK nach einem der Ansprüche 1 bis 3, **dadurch gekennzeichnet, dass** der PK erhalten ist durch Mischung des/der Polysaccharidpolymeren, dem/des Eiweiß/en, der/den Säure/n, dem/n Tensid/en und ggf. Wirkstoffen und/oder Zusatzstoffen in wässriger Lösung, Versprühen der wässrigen Mischung ohne Anwendung von Porogenen, ausgewählt aus überkritischem oder aufgelöstem CO₂, mikrokristallinen Salzen, Mikropartikeln auf Basis von Metalloxiden, Karbonaten oder Phosphaten oder Eis-Kristallen, und thermische Trocknung und/oder Strahlungstrocknung ohne Anwendung von Gefriertrocknung.

5. Verfahren zur Herstellung eines PK gemäß einem der Ansprüche 1 bis 4, **dadurch gekennzeichnet, dass** man
a) eine wässrige Zubereitung, enthaltend Polymere, ausgewählt aus Chitosan oder deacetyliertem Chitosan mit mittlerem MW von 70 bis oder 200 kDa bis 2000 kDa sowie Eiweißen, ausgewählt aus Kollagen I, II, oder III oder Mischungen hiervon, Kollagenhydrolysaten oder Gelatine mit mittlerem MW von 20 kDa bis 60 kDa, oder Mischungen hiervon, in einer Gesamtkonzentration von 0,5% bis 10%, zusammen mit Zusatzstoffen, wahlweise ausgewählt aus kosmetischen und/oder pharmazeutischen Wirkstoffen, Hilfsstoffen, oder Mischungen hiervon, sowie dem oder den Tensiden, ausgewählt aus C₆ - C₂₂ -Alkylglucosiden, C₁₀-C₂₂-Alkylglycosiden, C₆-C₂₂-Alkylpolyglykosiden, Saccharose- C₆ -C₂₂-Alkylestern, in einer Konzentration von 0,1 bis 5 %, sowie der oder den Säuren, ausgewählt aus Essigsäure, Propionsäure, Milchsäure, Apfelsäure, Bernsteinsäure, Malonsäure, Fumarsäure, Ascorbinsäure, Glutaminsäure, Salicylsäure, Pyrrolidoncarbonsäure oder Mischungen hiervon in einer Konzentration von 0,1 bis 5 %, in geeigneter Weise herstellt,
b) die wässrige Zubereitung aus a) mittels eines Schaumsprühers auf eine feste Oberfläche verschäumt und versprüht,
c) den in Schritt b) erhaltenen Schaum konventionell thermisch oder mittels Kontakt- oder Strahlungstrocknung oder Mischformen hiervon ohne Anwendung einer Gefriertrocknung trocknet; und
das getrocknete Produkt mit einer einstellbaren Dicke und einer flexibel eingestellten Porengröße im Bereich von 0,1 µm bis 1000 µm, vorzugsweise 10 µm bis 700 µm und mit wahlweise homogenen Porengrößenbereich erhält.

6. Verfahren nach Anspruch 5, **dadurch gekennzeichnet, dass** die Versprühung und Verschäumung in Schritt b) durch Liquid-Gas-Filtration einer mit Luft, einem Sauerstoff, Sauerstoffhaltigen Gas, Stickstoff, Argon oder Mischungen hiervon, ohne Anwendung von herkömmlichen Propellanten auf Fluor- und Kohlwasserstoff-Basis, einer aerolisierten Zubereitung aus Schritt a) erfolgt.

7. Verfahren nach Anspruch 5 oder 6, **dadurch gekennzeichnet, dass** die Versprühung in Schritt b) auf einer festen Oberfläche eines Materials biologischen und nicht biologischen Ursprungs, ausgewählt aus Polyethylen, Polypropylen, Polyester, PET, Edelstahl, Viskose, Zellulose, Hautgewebe, Hautoberfläche erfolgt.

8. Verfahren nach einem der Ansprüche 5 bis 7, **dadurch gekennzeichnet, dass** das Produkt gemäß Schritt c) in einem weiteren Schritt mit einem Vernetzer, gewählt aus Glutardialdehyd, Tannin, Carbodiimid, Methylenblau, Cumarin, Riboflavin oder Bengal Rose vernetzt wird.

9. Verfahren nach einem der Ansprüche 5 bis 8, **dadurch gekennzeichnet, dass** das Produkt gemäß Schritt c) in einem weiteren Schritt mit Lösungsmitteln gereinigt, sterilisiert und/oder konfektioniert wird.

10. Verfahren nach einem der Ansprüche 5 bis 9, **dadurch gekennzeichnet, dass** das erhaltene Produkt gemäß Schritt c) mit Wasser oder einer wässrigen Lösung zu hochporösen bioverträglichen stabilen 3 D-Hydrogelen hydratisiert wird.

11. Verfahren nach einem der Ansprüche 5 bis 10, **dadurch gekennzeichnet, dass** ein oder mehrere pharmazeutische Wirkstoffe, ausgewählt aus Antiallergenen, Antibiotika, Antiseptika, Entzündungshemmer, Immunsupressiva, Immunmodulatoren, Zytostatika, Immunstimulantien, Lokalanästetika, Hormonen, Antischmerzmittel, antineoplastischen Wirkstoffen, Zell- und Zellorgane regulierenden/unterstützenden Wirkstoffen (Wachstumsfaktoren, Zytokine), Wundheilenden Stoffe oder Mischungen hiervon vorliegen.

12. Poröser Körper (PK) gemäß einem der Ansprüche 1 bis 4 oder hergestellt nach einem der Ansprüche 5 bis 11 zur Anwendung oder in situ Direktanwendung bei der therapeutisch/dermatologischen Behandlung von beeinträchtigter, irritierter, infizierter, degenerierter Haut/Schleimhaut, Nägeln und Haaren, Haut- und Fußpilz, rheumatischer Arthritis, Schmerzlinderung, Psoriasis, Akne, Dermatitis, Neurodermitis, Hautumoren, Allopezia, chronischen Wunden, infizierten Wunden, Verbrennungen, Zahnkunde, Prothetisierung.

13. Verwendung eines porösen Körpers (PK) gemäß einem der Ansprüche 1 bis 2 oder hergestellt nach einem der Ansprüche 6 bis 12 zur kosmetischen topischen Behandlung oder in situ Direktanwendung von Altershaut, Cellulite, Narben, empfindlicher oder irritierter Haut.

14. Verwendung eines porösen Körpers (PK) gemäß einem der Ansprüche 1 bis 4 oder hergestellt nach einem der Ansprüche 5 bis 11 als Gerüst für die Kultivierung von eukaryotischen Zellen oder Stammzellen oder als Implantat.

## Claims

1. Porous body (PK), comprising 50 wt.% to 90 wt.% of a mixture of polysaccharid natural polymers, selected from chitosan, deacetylated chitosan with a mean MW of 70 kDa to 2000 kDa, in particular 200 kDa to 2000 kDa, as well as proteins, selected from collagen I, II, or III or mixtures thereof, collagen hydrolysate or gelatine with mean MW of 20 kDa to 60 kDa, or mixtures thereof, with furthermore 0.5 to 10 wt.% of at least one mono-/polycarboxylic acid, selected from acetic acid, propionic acid, lactic acid, malic acid, succinic acid, malonic acid, fumaric acid, ascorbic acid, glutamic acid, salicylic acid, pyrrolidone carboxylic acid or mixtures thereof, as well as 1.0 to 15 wt.% of one or several nonionic surfactants, selected from C₆ - C₂₂ - alkyl glucosides, C₁₀-C₂₂-alkyl glycosides, C₆-C₂₂-alkyl polyglycosides, sucrose-C₆ -C₂₂-alkyl esters, 0 bis 30 wt.% additives, optionally selected from excipients and active agents, 8 to 15 wt.% water or residual moisture, wherein the highly porous body has hexagonal or oval pore structures as well as flexibly adjustable pore sizes in the total range of 0.1 µm to 1000 µm, preferably 10 µm to 700 µm, with homogeneous pore distribution and has no pore structures generated by porogens, selected from supercritical or dissolved CO₂, microcrystalline salts, microparticles based on metal oxides, carbonates or phosphates or ice crystals and/or no lamellar structures.

2. PK according to claim 1, **characterized in that** pharmaceutical and/or cosmetic active agents and excipients are present as additives.

3. PK according to one of claims 1 or 2, **characterized in that** the PK is cross-linked with a cross-linking agent, selected from bifunctional covalent chemical cross-linking agents, glutardialdehyde, carbodiimide or from photochemical cross-linking agents methylene blue, coumarin, riboflavin or rose bengale.

4. PK according to one of claims 1 to 3, **characterized in that** the PK is generated by mixing the polysaccharid polymer/s, the protein/s, the acid/s, the surfactant/s and if applicable active agents and/or additives in aqueous solution, spraying of the aqueous mixture without the application of porogens, selected from supercritical or dissolved CO₂, microcrystallin salts, microparticles based on metal oxides, carbonates or phosphates or ice crystals, and thermal drying and/or radiation drying, without the application of freeze drying.

5. Method for the preparation of a PK according to one of claims 1 to 4, **characterized in that** one
a) prepares in a suitable manner an aqueous formulation, containing polymers selected from chitosan or deacetylated chitosan with a mean MW of 70 to or 200 kDa to 2000 kDa, as well as proteins, selected from collagen I, II, or III or mixtures thereof, collagen hydrolysate or gelatine with mean MW of 20 kDa to 60 kDa, or mixtures thereof, in a total concentration of 0.5% to 10%, together with additives optionally selected from cosmetic and/or pharmaceutical active agents, excipients or mixtures thereof, as well as the surfactant or the surfactants, selected from C₆ - C₂₂ - alkyl glucosides, C₁₀-C₂₂-alkyl glycosides, C₆-C₂₂-alkyl polyglycosides, sucrose-C₆ -C₂₂-alkyl esters, in a concentration of 0.1 to 5 %, as well as the acid or the acids, selected from acetic acid, propionic acid, lactic acid, malic acid, succinic acid, malonic acid, fumaric acid, ascorbic acid, glutamic acid, salicylic acid, pyrrolidone carboxylic acid or mixtures thereof in a concentration of 0.1 to 5 %,
b) foams and sprays the aqueous formulation of a) onto a solid surface by means of a foam sprayer,
c) dries the foam retrieved in step b) conventionally thermally or by means of contact or radiation drying or mixed forms thereof without application of freeze-drying, and yields the dried product with an adjustable thickness and a flexibly adjusted pore size in the range of 0.1 µm to 1000 µm, preferably 10 µm to 700 µm and with optionally homogenous pore size area.

6. Method according to claim 5, **characterized in that** the spraying and foaming in step b) takes place by liquid-gas filtration of a formulation of step a) aerosolized with air, an oxygen, oxygen containing gas, nitrogen, argon or mixtures thereof, without application of common propellants based on fluorine and hydrocarbon.

7. Method according to claim 5 or 6, **characterized in that** the spraying in step b) takes place onto a solid surface of a material of biological or nonbiological origin, selected from polyethylene, polypropylene, polyester, PET, special steel, viscose, cellulose, skin tissue, skin surface.

8. Method according to one of claims 5 to 7 **characterized in that** the product according to step c) is cross-linked in a futher step with a cross-linking agent selected from glutardialdehyde, tannin, carbodiimid, methylen blue, coumarin, riboflavin or rose bengale.

9. Method according to one of claims 5 to 8, **characterized in that** the product according to step c) in a further step is cleaned with solvents, sterilized and/or tailored.

10. Method according to one of claims 5 to 9, **characterized in that** the retrieved product according to step c) is hydrated with water or an aqueous solution to highly porous biocompatible stable 3 D hydrogels.

11. Method according to one of claims 5 to 10, **characterized in that** one or more pharmaceutical active agents are present, selected from antiallergens, antibiotics, antiseptics, anti-inflammatories, immunosupressants, immunomodulators, cytostatics, immunostimulants, local anesthetics, hormons, analgesics, antineoplastic active agents, cell- and cell organs regulating/supporting active agents (growth factors, cytokines), wound healing substances or mixtures thereof.

12. Porous body (PK) according to one of claims 1 to 4 or prepared according to one of claims 5 to 11 for application or in situ direct application in the therapeutical/dermatological treatment of impaired, irritated, infected, degenerated skin/mucous membrane, nails and hair, skin and foot fungus, rheumatoid arthritis, pain relief, psoriasis, acne, dermatitis, neurodermatitis, skin tumors, allopezia, chronic wounds, infected wounds, burns, dentistry, prosthetics.

13. Use of a porous body (PK) according to one of claims 1 to 2 or prepared according to one of claims 6 to 12 for cosmetic topical treatment or in situ direct application of aged skin, cellulite, scars, sensitive or irritated skin.

14. Use of a porous body (PK) according to one of claims 1 to 4 or prepared according to one of claims 5 to 11 as scaffold for the cultivation of eukaryotic cells or stem cells, or as an implant.

## Revendications

1. Corps poreux (PK) comprenant de 50 % à 90 % en poids d'un mélange de polymères naturels polysaccharidiques choisis parmi le chitosane, le chitosane désacétylé ayant un poids moléculaire moyen allant de 70 kDa à 2000 kDa, en particulier de 200 kDa à 2000 kDa, ainsi que des protéines choisies parmi le collagène I, Il ou III ou des mélanges de ceux-ci, l'hydrolysat de collagène ou la gélatine ayant un poids moléculaire moyen de 20 kDa à 60 kDa, ou des mélanges de ceux-ci, comprenant en outre 0,5 à 10 % en poids d'au moins un acide mono-/polycarboxylique choisi parmi l'acide acétique, l'acide propionique, l'acide lactique, l'acide malique, l'acide succinique, l'acide malonique, l'acide fumarique, l'acide ascorbique, l'acide glutamique, l'acide salicylique, l'acide pyrrolidone-carboxylique ou les mélanges de ceux-ci, ainsi que de 1,0 à 15 % en poids d'un ou plusieurs tensioactifs non ioniques choisis parmi les alkylglucosides en C₆-C₂₂, les alkylglycosides en C₁₀-C₂₂, les alkylpolyglycosides en C₆-C₂₂, les esters d'alkyle en C₆-C₂₂ du saccharose, 0 à 30 % en poids d'additifs, choisis au choix parmi des adjuvants et des substances actives, 8 à 15 % en poids d'eau ou d'humidité résiduelle, le corps hautement poreux présentant des structures poreuses hexagonales ou ovales, ainsi que des tailles de pores réglables de manière flexible dans une gamme totale allant de 0,1 µm à 1000 µm, de préférence de 10 µm à 700 µm, avec une répartition homogène des pores et sans structures poreuses générées par des porogènes choisis parmi le CO₂ supercritique ou dissous, les sels microcristallins, les microparticules à base d'oxydes métalliques, de carbonates ou de phosphates ou de cristaux de glace et/ou sans structures lamellaires.

2. PK selon la revendication 1, **caractérisé en ce qu'**il contient, en tant qu'additifs, des substances actives et auxiliaires pharmaceutiques et/ou cosmétiques.

3. PK selon l'une des revendications 1 ou 2, **caractérisé en ce que** le PK est réticulé au moyen d'un agent de réticulation choisi parmi les agents de réticulation chimiques covalents bifonctionnels, le glutardialdéhyde, le carbodiimide ou parmi les agents de réticulation photochimiques le bleu de méthylène, la coumarine, la riboflavine ou le rose de Bengale.

4. PK selon l'une des revendications 1 à 3, **caractérisé en ce que** le PK est obtenu par mélange du ou des polymères polysaccharidiques, de la ou des protéines, du ou des acides, du ou des tensioactifs et, le cas échéant, des substances actives et/ou des additifs en solution aqueuse, par pulvérisation du mélange aqueux sans utilisation de porogènes, choisis parmi le CO₂ supercritique ou dissous, les sels microcristallins, les microparticules à base d'oxydes métalliques, de carbonates ou de phosphates ou de cristaux de glace, et par séchage thermique et/ou séchage par rayonnement sans utilisation de lyophilisation.

5. Procédé de fabrication d'un PK selon l'une des revendications 1 à 4, **caractérisé par**
a) une préparation aqueuse contenant des polymères choisis parmi le chitosane ou le chitosane désacétylé ayant un poids moléculaire moyen allant de 70 à 200 kDa à 2000 kDa, ainsi que des protéines choisies parmi le collagène I, II, ou III ou des mélanges de ceux-ci, des hydrolysats de collagène ou de la gélatine ayant un poids moléculaire moyen allant de 20 kDa à 60 kDa, ou les mélanges de ceux-ci, à une concentration totale allant de 0,5 % à 10 %, ainsi que des additifs choisis optionnellement parmi des principes actifs cosmétiques et/ou pharmaceutiques, des excipients ou les mélanges de ceux-ci, un ou des tensioactifs choisis parmi les alkylglucosides en C₆-C₂₂, les alkylglycosides en C₁₀-C₂₂, les alkylpolyglycosides en C₆-C₂₂, des esters d'alkyle en C₆-C₂₂ du saccharose, en une concentration allant de 0,1 à 5 %, ainsi qu'un ou des acides choisis parmi l'acide acétique, l'acide propionique, l'acide lactique, l'acide malique, l'acide succinique, l'acide malonique, l'acide fumarique, l'acide ascorbique, l'acide glutamique, l'acide salicylique, l'acide pyrrolidone-carboxylique ou les mélanges de ceux-ci, en une concentration allant de 0,1 à 5 %, de manière appropriée,
b) le fait que la préparation aqueuse issue de l'étape a) est transformée en mousse et pulvérisée sur une surface solide au moyen d'un pulvérisateur à mousse
c) le fait que la mousse obtenue à l'étape b) est séchée de manière conventionnelle par voie thermique ou par séchage par contact ou par rayonnement ou par des formes mixtes de ceux-ci sans utilisation de lyophilisation ; et
il est obtenu le produit séché ayant une épaisseur réglable et une taille de pores réglée de manière flexible dans la gamme allant de 0,1 µm à 1000 µm, de préférence de 10 µm à 700 µm, et avec une plage de tailles de pores homogènes au choix.

6. Procédé selon la revendication 5, **caractérisé en ce que** la pulvérisation et le moussage à l'étape b) sont effectués par filtration liquide-gaz d'une préparation aérosolisée de l'étape a) avec de l'air, de l'oxygène, un gaz contenant de l'oxygène, de l'azote, de l'argon ou des mélanges de ceux-ci, sans utilisation de propulseurs classiques à base de fluor et d'hydrocarbures.

7. Procédé selon la revendication 5 ou la revendication 6, **caractérisé en ce que** la pulvérisation à l'étape b) est effectuée sur une surface solide d'un matériau d'origine biologique ou non-biologique, choisi parmi le polyéthylène, le polypropylène, le polyester, le PET, l'acier inoxydable, la viscose, la cellulose, le tissu cutané, la surface cutanée.

8. Procédé selon l'une des revendications 5 à 7, **caractérisé en ce que** le produit selon l'étape c) est, dans une étape supplémentaire, réticulé au moyen d'un agent de réticulation choisi parmi le glutardialdéhyde, le tanin, le carbodiimide, le bleu de méthylène, la coumarine, la riboflavine ou le rose de Bengale.

9. Procédé selon l'une des revendications 5 à 8, **caractérisé en ce que** le produit selon l'étape c) est, dans une étape supplémentaire, nettoyé, stérilisé et/ou conditionné au moyen de solvants.

10. Procédé selon l'une des revendications 5 à 9, **caractérisé en ce que** le produit obtenu selon l'étape c) est hydraté avec de l'eau ou une solution aqueuse pour former des hydrogels 3D stables, biocompatibles et hautement poreux.

11. Procédé selon l'une des revendications 5 à 10, **caractérisé en ce que** sont utilisés un ou plusieurs principes actifs pharmaceutiques, choisis parmi les antiallergènes, les antibiotiques, les antiseptiques, les anti-inflammatoires, les immunosuppresseurs, les immunomodulateurs, les cytostatiques, les immunostimulants, les anesthésiques locaux, les hormones, les analgésiques, les agents antinéoplasiques, les agents régulateurs/soutiens des cellules et des organes cellulaires (facteurs de croissance, cytokines), les substances cicatrisantes, ou des mélanges de ceux-ci.

12. Corps poreux (PK) selon l'une des revendications 1 à 4, ou fabriqué selon l'une des revendications 5 à 11, pour une utilisation ou une application directe in situ dans le traitement thérapeutique/dermatologique de la peau/muqueuse, des ongles et des cheveux abîmés, irrités, infectés, dégénérés, des mycoses cutanées et fongiques, de l'arthrite rhumatoïde, pour le soulagement de la douleur, du psoriasis, de l'acné, de la dermatite, de la neurodermite, des tumeurs cutanées, de l'alopécie, des plaies chroniques, des plaies infectées, des brûlures, de la dentisterie, des prothèses.

13. Utilisation d'un corps poreux (PK) selon l'une des revendications 1 à 2, ou fabriqué selon l'une des revendications 6 à 12, pour le traitement cosmétique topique ou l'application directe in situ de la peau vieillissante, de la cellulite, des cicatrices, de la peau sensible ou irritée.

14. Utilisation d'un corps poreux (PK) selon l'une des revendications 1 à 4, ou fabriqué selon l'une des revendications 5 à 11, en tant que support pour la culture de cellules eucaryotes ou de cellules souches, ou en tant qu'implant.
